# EUROPEAN PATENT SPECIFICATION

(11) **EP 1 553 923 B1**
(45) Date of publication and mention of the grant of the patent: **09.09.2009**
(21) Application number: 03761518.4
(22) Date of filing: 26.06.2003
(51) Int. Cl.: A61K 9/127, A61K 31/47, A61P 35/00

(54) **CAMPTOTHECIN-CARBOXYLATE FORMULATIONS**
CAMPTOTHECIN-CARBOXYLAT-FORMULIERUNGEN
FORMULATIONS DE CAMPTOTH CINE-CARBOXYLATE

(30) Priority: 26.06.2002 US 391244 P; 26.06.2002 US 391246 P; 23.08.2002 EP 02018907
(43) Date of publication of application: 20.07.2005
(73) Proprietor: MediGene AG, 82152 Planegg/Martinsried (DE)
(72) Inventor: HAAS, Heinrich, 80337 München (DE); SCHULZE, Brita, 82432 Walchensee (DE); MICHAELIS, Uwe, 82362 Weilheim (DE); TEIFEL, Michael, 64331 Weiterstadt (DE); SAUER, Birgitta, 82166 Gräfelfing (DE); FICHERT, Thomas, 66606 St. Wendel (DE)
(74) Representative: Weiss, Wolfgang
(86) International application number: PCT/EP2003/006760
(87) International publication number: WO 2004/002454

(56) References cited:
- EP-A- 0 538 534
- WO-A-95/08986
- WO-A-99/13816
- EMERSON D L: "Liposomal delivery of camptothecins" PHARMACEUTICAL SCIENCE AND TECHNOLOGY TODAY, ELSEVIER TRENDS JOURNALS, CAMBRIDGE,, GB, vol. 3, no. 6, 1 June 2000 (2000-06-01), pages 205-209, XP002235822 ISSN: 1461-5347
- LUNDBERG B B: "BIOLOGICALLY ACTIVE CAMPTOTHECIN DERIVATIVES FOR INCORPORATION INTO LIPOSOME BILAYERS AND LIPID EMULSIONS" ANTI-CANCER DRUG DESIGN, BASINGSTOKE, GB, vol. 13, no. 5, 1998, pages 453-461, XP000878681 ISSN: 0266-9536

## Description

The present invention relates to compositions, particularly colloidal nanoaggregates comprising the carboxylate form of a campothecin drug associated with at least one catyonic amphiphile and/or cationic polymer, which has a positive net charge. The composition or nanoaggregate may be present as an emulsion, droplet, micelle, liposome, nanoparticle or nanocapsule.

Camptothecin (CPT) is a quinoline-based alkaloid, which can be isolated from the Chinese tree Camptotheca acuminata (Wall, Wani et al. 1966). It was first described and tested as an anti-cancer drug in the 60ies and 70ies. Anti-tumor activity was noted in animal models and in clinical studies. However, patients experienced severe side reactions such as neutropenia, thrombocytopenia, haemorrhagic cystitis (Wall and Wani 1996). The therapeutic effect of camptothecin in humans had been questioned (Moertel, Schutt et al. 1972; Muggia, Creaven et al. 1972). It continued to be of high interest as a potential candidate for the development of an anti-cancer drug, and it was found that it has a particular mode of action, wherein binding to the topoisomerase I-DNA complex induces DNA breaks and cell death (topoisomerase I inhibitor) (Hsiang and Liu 1988).

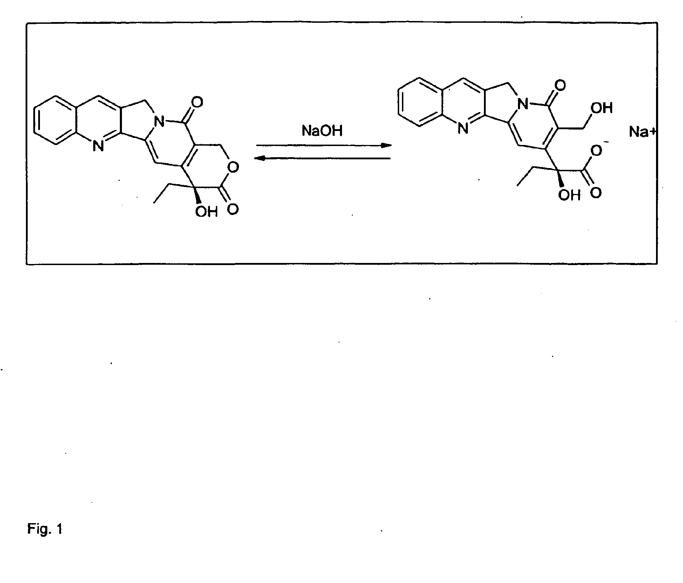

A fundamental molecular property of CPT is its pH dependent equilibrium between the lactone and the carboxylate form (Fig. 1). The lactone form is lipophilic, while the carboxylate, which predominates at physiological pH and above, is water-soluble. Since the lactone form is too lipophilic to be administered without difficulties, initially, CPT was transformed into the water-soluble sodium salt (NCS 100880). However, due to unacceptable side reactions, the development of that compound was not further pursued (Moertel, Schutt et al. 1972; Muggia, Creaven et al. 1972).

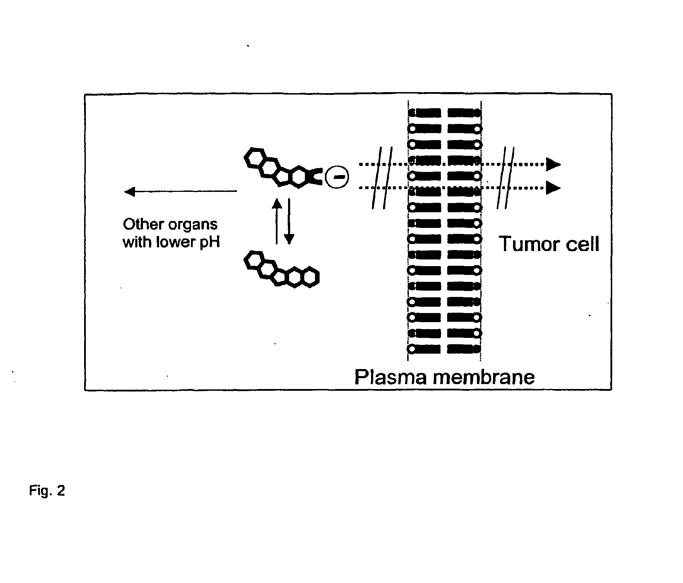

In subsequent research, the equilibrium between the lactone form and the carboxylate form was found fundamental for the cytostatic activity and the appearance of side effects within anti-cancer treatment: CPT-carboxylate was identified as being responsible for the observed side reactions and it was considered to be significantly less active than CPT-lactone (Hertzberg, et. al. 1989). The lower membrane permeability of the carboxylate form compared to that of the lactone, and the shift of the molecular equilibrium as a function of the pH value at the respective local physiological environment were considered to be involved in these activity differences (see Fig. 2).

Due to these detrimental properties of CPT-carboxylate, further efforts for the development of CPT based drugs were concentrated on the manufacture of formulations which contain the lactone form and which are free of the carboxylate form. This is especially important, since under physiological conditions, the carboxylate is the predominant molecular form of CPT, and the lactone form hydrolyses to the carboxylate within a short period of time (typically few hours or less). Therefore, a main objective of the development of CPT drugs was to stabilize the lactone form and to find ways to administer it without difficulties (Zunino et al. 2002).

Various strategies have been followed to stabilize the lactone ring and to concomitantly improve the solubility properties of the molecule in order to provide easier administration. Particularly, functionalisation of the original molecule to different types of derivatives, synthesis of prodrugs, and several methods of administration have been pursued (Kehrer, Soepenberg et al. 2001). However, none of them has brought the desired results of resolving the above described inherent difficulties of camptothecin as an anti-cancer drug.

In another approach, liposomes were used to protect CPT-lactone from hydrolysis. This was realized by encapsulation of CPT in a liposome under acidic conditions, or by embedding CPT into the lipid bilayer of a liposome in order to protect the lactone form from hydrolysis and from blood and serum interactions. In fact, by embedding CPT-lactone in the hydrophobic region of the vesicular lipid bilayer (US 5552156) the lactone form was not exposed to the aqueous environment and hydrolysis was significantly slowed down. However, only very low drug/lipid ratios could be achieved and therefore the necessary dosages for clinical use could not be realized.

In a further liposome-based approach, CPT-lactone was embedded into the lipid bilayer of a liposome comprising phospholipids, which contain unsaturated fatty acids (US 5834012). Thereby a stabilization effect was reported. It was proposed that the latter was due to the interaction of CPT in the lactone form with the unsaturated fatty acid chains of the lipids.

However, as with other approaches, up to now no substantial breakthrough towards a functional CPT drug formulation could be achieved.

Thus, the problem underlying the present invention was to provide a composition comprising a camptothecin drug and a formulation thereof for most efficient delivery to a subject in need under reduction of side effects.

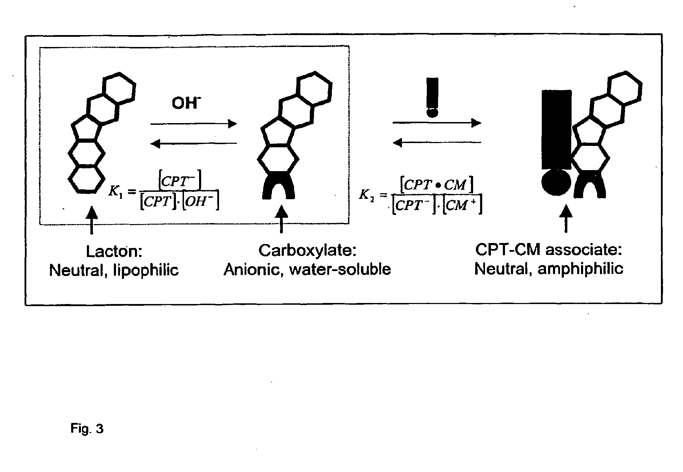

The solution to the above problem is achieved according to the invention by providing the embodiments characterized in the claims. The invention relates to a composition comprising the carboxylate form of a camptothecin drug associated with at least one cationic amphiphile and/or cationic polymer (Fig. 3) which comprises a positively charged (cationic) group, preferably a cationic amphiphile, wherein said composition has a molar ratio of at least about 1:1 of the organic cationic molecule (CM) to camptothecin carboxylate and is substantially free of the lactone form of said drug.

In the context of the present invention "camptothecin drug" refers to camptothecin itself or a derivative thereof. A camptothecin derivative is obtained by any chemical derivatization of camptothecin (see structure). A non-limiting list of possible camptothecin drugs is given under: http://dtp.nci.nih.gov as from Aug. 19, 2002. In the sketch of the molecule, the most frequent derivatization sites are outlined as R₁-R₅.

Structure of camptothecin drugs:

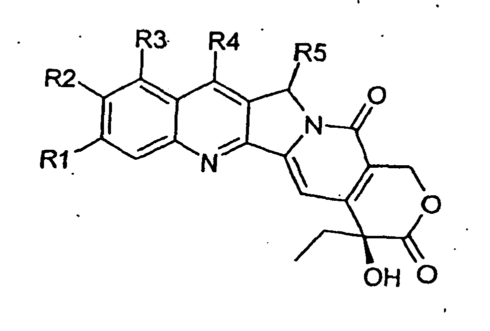

In the following table, typical examples for derivatization at different sites are listed. Camptothecin may be present as a hydrochloride. The lactone ring (E-ring) may be seven-membered instead of six-membered (homocamptothecins).

| **Name** | **R1** | **R2** | **R3** | **R4** | **R5** |
|---|---|---|---|---|---|
| Camptothecin | H | H | H | H | H |
| 9-Nitro-camptothecin | H | H | NO₂ | H | H |
| 9-Amino-camptothecin | H | H | NH₂ | H | H |
| 10-Hydroxy-camptothecin | H | OH | H | H | H |
| Topotecan | H | OH | -CH₂-N- (CH₃)₂ | H | H |
| SN38 | H | OH | H | CH₂-CH₃ | H |
| Camptosar^{®} (Irinotecan) | H | 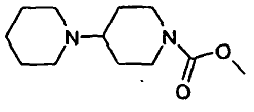 | H | CH₂-CH₃ | H |
| Lurtotecan^{®} | R1 and R2 is: O-CH2-CH2-O | | H | H | H |
| DX-8951f | F | CH₃ | R₃ and R₄ is: -CH2-CH2-CH(NH₂)- | | H |

Derivatization can influence the properties of CPT to make the molecule more hydrophilic or more lipophilic, or that the lactone-carboxylate equilibrium is affected. In the context of the application of CPT as an anti-cancer drug, derivatization is intended to maintain or to increase activity.

For producing the inventive composition, camptothecin or any suitable camptothecin derivative in the carboxylate form is provided. For most efficient preparation, the camptothecin drug is provided as a camptothecin carboxylate salt. The binding efficacy of a given camptothecin derivative to the organic counter molecule can be higher or less high with respect to camptothecin. Suitable residues in the R₁-R₄ position can be favourable for the binding to an amphiphilic counter molecule.

Subsequently the invention will be explained in detail with camptothecin as working example, however, the illustration also relates to any camptothecin derivative.

The inventive composition is a new entity comprising the carboxylate form of a camptothecin drug associated with at least one cationic amphiphile and/or cationic polymer having a positive net charge , wherein the molar ratio of the charged groups in the entity is up to about 2:1, preferably up to about 1.5:1, more preferably up to about 1.1:1 and most preferably about 1:1 of the organic cationic amphiphilic or cationic polymer with respect to the carboxylate form of a camptothecin drug. The composition is substantially free of the lactone form of the camptothecin drug. The inventive composition contains a new entity, constituting a new molecular composition with unique physico-chemical properties.

The cationic polymer with a positive net charge can be any cationic polyelectrolyte, such as polyallylamine or polyethyleneimine, as well as polyamino acids or polysaccharides. The molecular weight is preferably between about 5 and 500 kDa. The CPT binds to the polymer by simple mixing. The resulting composition can be used as a constituent for the self assembly of different types of nanoaggregates (nanoparticles, nanocapsules).

The cationic amphiphile with a positive net charge can be monovalent or polyvalent. It may be selected from lipids, lysolipids or pegylated lipids with a positive net charge. Useful cationic lipids thereby include:
DDAB, dimethyldioctadecyl ammonium bromide;
N-[1-(2,3-dioleoyloxy)propyl]-N,N,N-trimethyl ammonium methylsulfate (DOTAP); 1,2-diacyloxy-3-trimethylammonium propanes, (including but not limited to: dioleoyl, dimyristoyl, dilauroyl, dipalmitoyl and distearoyl; also two different acyl chains can be linked to the glycerol backbone); N-[1-(2,3-dioleoyloxy)propyl]-N,N-dimethyl amine (DODAP); 1, 2-diacyloxy-3-dimethylammonium propanes, (including but not limited to: dioleoyl, dimyristoyl, dilauroyl, dipalmitoyl and distearoyl; also two different acyl chain can be linked to the glycerol backbone); N-[1-(2,3-dioleyloxy)propyl]-N,N,N-trimethylammonium chloride (DOTMA); 1,2-dialkyloxy-3-dimethylammonium propanes, (including but not limited to: dioleyl, dimyristyl, dilauryl, dipalmityl and distearyl; also two different alkyl chain can be linked to the glycerol backbone); dioctadecylamidoglycylspermine (DOGS);,
3β-[N-(N',N'-dimethylaminoethane)carbamoyl]cholesterol (DC-Chol); 2,3-dioleoyloxy-N-(2-(sperminecarboxamido)-ethyl)-N,N-dimethyl-1-propa naminium trifluoro-acetate (DOSPA); β-alanyl cholesterol; cetyl trimethyl ammonium bromide (CTAB); diC14-amidine;
N-tert-butyl-N'-tetradecyl-3-tetradecylaminopropionamidine; 14Dea2; N-(alpha-trimethylammonioacetyl)didodecyl-D-glutamate chloride (TMAG); O,O'-ditetradecanoyl-N-(trimethylammonioacetyl)diethanolamine chloride; 1,3-dioleoyloxy-2-(6-carboxy-spermyl)-propytamide (DOSPER); N,N,N',N'-tetramethyl-N,N'-bis(2-hydroxylethyl)-2,3-dioleoyloxy-1,4-butanediammonium iodide;
1-(2-(acyloxy)ethyl]2-alkyl(alkenyl)-3-(2-hydroxyethyl)-imidazolinium chloride derivatives as described by Solodin et al. (1995) Biochem. 43:13537-13544, such as 1-[2-(9(Z)-octadecenoyloxy)ethyl]-2-(8(Z)-heptadecenyl-3-(2-hydroxyethyl)imidazolinium chloride (DOTIM), 1-[2-(hexadecanoyloxy)ethyl]-2-pentadecyl-3-(2-hydroxyethyl)imidazolinium chloride (DPTIM), 2,3-dialkyloxypropyl quaternary ammonium compound derivatives, contain? a hydroxyalkyl moiety on the quaternary amine, as described e.g. by Felgner et al. [Felgner et al. J. Biol. Chem. 1994, 269, 2550-2561] such as: 1,2-dioleoyl-3-dimethyl-hydroxyethyl ammonium bromide (DORI), 1,2-dioleyloxypropyl-3-dimethyl-hydroxyethyl ammonium bromide (DORIE), 1,2-dioleyloxypropyl-3-dimetyl-hydroxypropylammonium bromide (DORIE-HP), 1,2-dioleyloxypropyl-3-dimethyl-hydroxybutyl ammonium bromide (DORIE-HB),
1,2-dioleyloxypropyl-3-dimethyl-hydroxypentyl ammonium bromide (DORIE-Hpe),1,2-dimyristyloxypropyl-3-dimethyl-hydroxylethylammonium bromide (DMRIE), 1,2-dipalmityloxypropyl-3-dimethyl-hydroxyethyl ammoniumbromide(DPRIE),1,2-disteryloxypropyl-3-dimethyl-hydroxyethyl ammonium bromide (DSRIE); cationic esters of acyl carnitines as reported by Santaniello et al. [US5498633]; cationic triesters of phospatidylcholine, i.e., 1,2-diacyl-sn-glycerol-3-ethylphosphocholines, where the acyl chains can be saturated or unsaturated and branched or non-branched with a chain length from C₁₂ to C₂₂, the two acyl chains being not necessarily identical.

In a preferred embodiment the cationic amphiphile is selected from a quaternary ammonium compound such as N-[1-(2,3-diacyloxy)propyl]-N,N,N-trimethyl ammonium, which may be provided as a salt with a pharmaceutically acceptable counter anion which may be selected from the group consisting of chloride, bromide, fluoride, iodide, nitrate, sulfate, methyl sulfate, phosphate, acetate, benzoate, citrate, glutamate or lactate.

The inventive composition is characterized in that the carboxylate form of a camptothecin drug is associated with an cationic amphipile and/or cationic polymer. The association is driven by electrostatic forces and optionally amphiphilic interactions. Herein, the term CM-CPT is used to denominate the composition comprising a camptothecin drug and the cationic counter molecule. The composition may be obtained by adding CPT-carboxylate to the counter ion, or vice-versa, in any suitable way. Detailed procedures for preparing the inventive composition are given in subsequent sections.

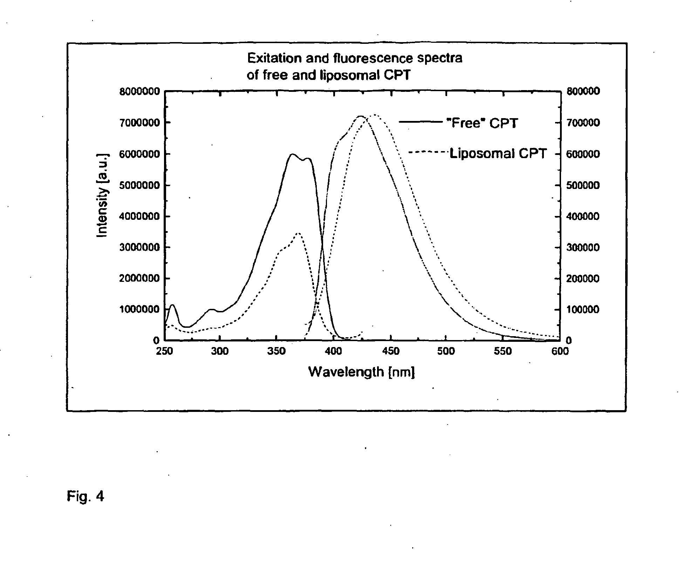

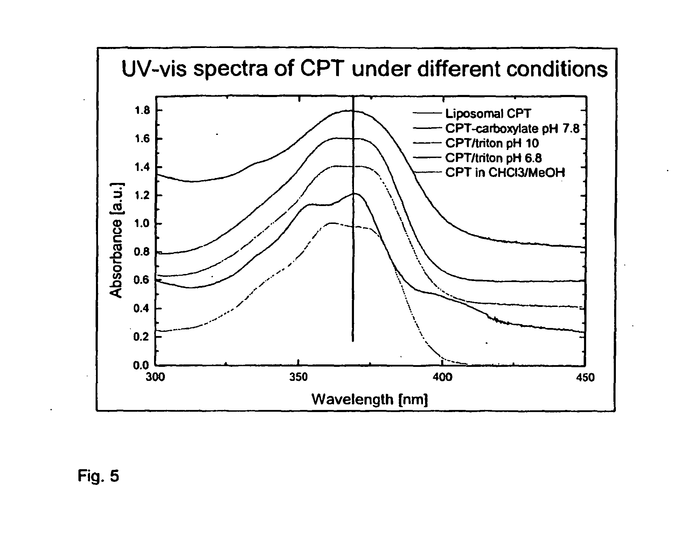

The presence of the CM-CPT can be shown in situ, for example by spectroscopic methods, since the spectra of the CPT-carboxylate, CPT-lactone and CM-CPT are clearly different (Fig. 4, Fig. 5). In Fig. 4, the fluorescence excitation and emission spectra of free CPT in organic solvent and CM-CPT as an liposomal formulation in aqueous environment are given. In Fig. 5 the UV-vis spectra of CM-CPT as a liposomal formulation, CPT in aqueous environment under various conditions and CPT-lactone in organic solvent are compared.

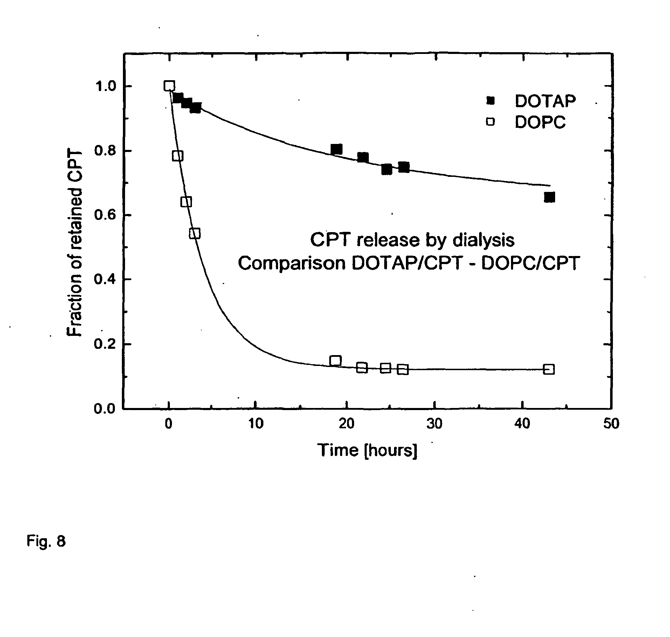

The binding selectivity of CPT to cationic molecules is clearly demonstrated in Fig. 8, where the binding of CPT to DOPC (a neutral lipid) and to DOTAP (a cationic lipid) in aqueous suspension is compared. Therein, ethanol injections of the lipid solutions into aqueous solutions of CPT-carboxylate were performed, and the resulting colloidal suspensions were dialyzed against a large excess of buffer in order to remove the remaining free CPT-carboxylate (experimental details are given in the following section). The release of CPT-carboxylate as a function of time was determined by UV-vis spectroscopy. As can be seen, for DOPC virtually all of the CPT was released from the suspension, i.e., no binding to DOPC could be determined. On the contrary, with DOTAP an equilibrium was reached where the majority of the CPT remained bound to the lipid. This demonstrates that CPT-carboxylate binds to DOTAP, but not to DOPC.

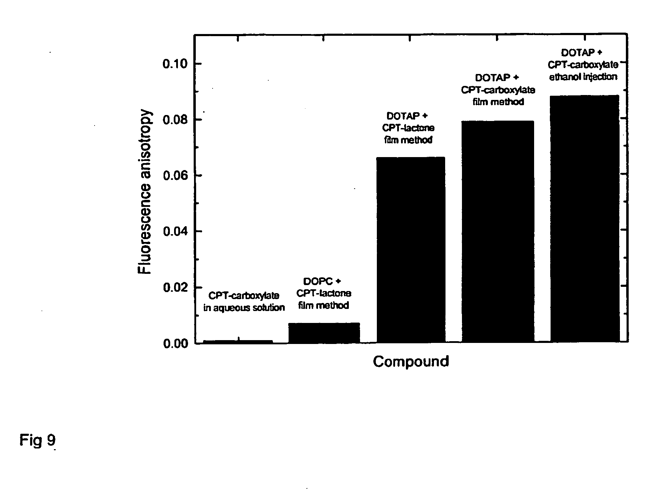

These findings are corroborated by fluorescence anisotropy measurements of the CPT. In this experiment, an increase of the fluorescence anisotropy is expected if the fluorophore is inserted into a liposome and thus its mobility is decreased. As can be seen in Fig. 9, with DOPC only a very small anisotropy, close to the one of the free CPT-carboxylate in aqueous solution, was measured, while with DOTAP, for all shown formulations, a much higher anisotropy was observed. These observations indicate, that only with cationic amphiphiles binding to CPT-carboxylate and formation of stable colloidal suspensions occurs. With DOPC, such a binding could not be observed. DOTAP and DOPC both comprise unsaturated fatty acid chains, but DOTAP is positively charged while DOPC is neutral. Therefore, the observations indicate, that the driving force for the binding of the negatively charged CPT-carboxylate is the positive charge of the lipid. The electrostatic interaction driven binding of CPT-carboxylate to cationic molecules is much more efficient that the hydrophobic interaction driven binding of CPT-lactone to the unsaturated fatty acid residues of lipids, which is described in US 5834012.

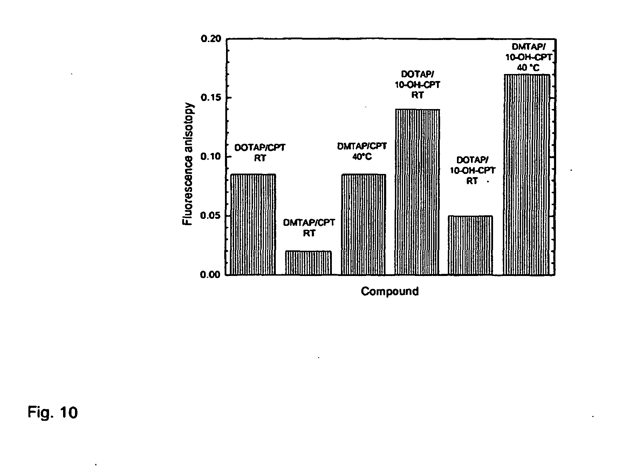

For efficient binding, in addition to charge, the phase state of the fatty acid chains is important. This is demonstrated in Fig. 10, where the results from fluorescence anisotropy measurements of CPT or 10 OH-CPT with DMTAP or DOTAP at room temperature and at 40°C are depicted. DOTAP has its main phase transition (Tₘ) near 37°C (Lewis, Tristram-Nagle et al. 2001). Below that temperature the fatty acid chains are in the gel phase (a crystalline like state) and above they are in the liquid crystalline (fluid-like) state. The data demonstrate that high anisotropy and thus strong binding was found at 40°C, i.e., above (Tₘ) in the fluid like state. This underlines, that for high binding efficacy a suitable phase state of the hydrocarbon chains is important. With any lipid which is in the suitable state, high binding efficacy can be realized, independently if the fatty acids are composed of oleic acids or not. Thus, the composition of the present invention preferably contains cationic amphiphile having alkyl chains in a fluid-like state. Further, these data demonstrate that binding of a CPT drug to a cationic molecule is not limited to CPT itself but is also applicable to any suitable CPT derivate wherein even higher binding efficacies may be obtained.

Unless defined otherwise, all technical and scientific terms used in this specification shall have the same meaning as commonly understood by persons of ordinary skill in the art to which the present invention pertains.

"About" in the context of amount values refers to an average deviation of maximum +/- 20 %, preferably +/- 10 % based on the indicated value. For example, an amount of about 30 mol% cationic lipid refers to 30 mol% +/- 6 mol% and preferably 30 mol% +/- 3 mol% cationic lipid with respect to the total lipid/amphiphile molarity.

"Amphiphile" refers to a molecule, which consists of a water-soluble (hydrophilic) and an oil-soluble (lipophilic) part. The lipophilic part preferably contains at least one alkyl chain having at least 10, preferably at least 12 carbon atoms.

"Associating" a negatively charged compound to a cationic amphiphile refers to binding said compound to the cationic organic molecule by electrostatic and/or amphipatic (or amphiphilic) forces. The resulting composition is preferably characterized by a charge ratio close to 1:1, but also ratios of 1.5:1 or > 2:1 may be used depending on the nature of the molecules.

"Camptothecin" refers to 20(S)-Camptothecine (1H-Pyrano[3',4':6,7] indolizino[1,2-b]quinoline-3,14 (4H,12H)-dione, 4-ethyl-4-hydroxy-,(S)-), **CAS 7689-03-4.**

"Cancer" refers to the more common forms of cancers such as bladder cancer, breast cancer, colorectal cancer, endometrial cancer, head and neck cancer, leukaemia, lung cancer, lymphoma, melanoma, non-small-cell lung cancer, ovarian cancer, prostate cancer and to childhood cancers such as brain stem glioma, cerebellar astrocytoma, cerebral astrocytoma, ependymoma, Ewing's sarcoma/family of tumors, germ cell tumor, extracranial, hodgkin's disease, leukemia, acute lymphoblastic, leukemia, acute myeloid, liver cancer, medulloblastoma, neuroblastoma, non-hodgkin's lymphoma, osteosarcoma/malignant fibrous histiocytoma of bone, retinoblastoma, rhabdomyosarcoma, soft tissue sarcoma, supratentorial primitive neuroectodermal and pineal tumors, unusual childhood cancers, visual pathway and hypothalamic glioma, Wilms' Tumor and other childhood kidney tumors and to less common cancers including acute lymphocytic leukaemia, adult acute myeloid leukaemia, adult non-hodgkin's lymphoma, brain tumor, cervical cancer, childhood cancers, childhood sarcoma, chronic lymphocytic leukaemia, chronic myeloid leukaemia, esophageal cancer, hairy cell leukaemia, kidney cancer, liver cancer, multiple myeloma, neuroblastoma, oral cancer, pancreatic cancer, primary central nervous system lymphoma, skin cancer, small-cell lung cancer.

"Carrier" refers to a diluent, adjuvant, excipient, or vehicle which is suitable for administering a diagnostic or therapeutic agent. The term also refers to a pharmaceutically acceptable component(s) that contains, complexes or is otherwise associated with an agent to facilitate the transport of such an agent to its intended target site. Carriers include those known in the art, such as liposomes, polymers, lipid complexes, serum albumin, antibodies, cyclodextrins and dextrans, chelates, or other supramolecular assemblies.

"Cationic" refers to an agent that has a net positive charge or positive zeta potential under the respective environmental conditions. In the present invention, it is referred to environments where the pH is in the range between 3 and 9, preferably between 5 and 8.

"Cationic amphiphile" or "cationic lipid" refers to encompass any amphiphile or lipid which has a positive charge. In the present invention, it is referred to environments where the pH is in the range between 3 and 9, preferably between 5 and 8.

"Cationic liposome" refers to a liposome which is positively charged. In the present invention, it is referred to environments where the pH is in the range between 3 and 9, preferably between 5 and 8. The cationic liposomes are prepared from the cationic lipids or amphiphiles themselves or in admixture with other amphiphiles, particularly neutral or anionic lipids.

"CM" is used in the text as an abbreviation for cationic molecule.

"Colloids" or colloidal particles refers to particles or molecular aggregates dispersed in a medium in which they are insoluble and have a size between about 5 nm and 5000 nm.

"Colloidal nanoaggregate having an overall positive charge" as used herein refers to a colloidal nanoaggregate comprising optionally the inventive composition and having an excess of positively charged molecules in the outer molecular layer.

"Cryoprotectant" refers to a substance that helps to protect a species from the effect of freezing.

"Derivative" refers to a compound derived from some other compound while maintaining its general structural features. Derivatives may be obtained for example by chemical functionalization or derivatization.

"Disease characterized by enhanced angiogenic activity" refers to processes such as tissue inflammation, arthritis, asthma, macular degeneration, tumor growth, and diabetic retinopathy.

"Drug" as used herein refers to a pharmaceutically acceptable pharmacologically active substance, physiologically active substances and/or substances for diagnosis use.

"Homogenization" refers to a physical process that achieves a uniform distribution between several components. One example is high-pressure homogenisation.

"Lipid" refers to its conventional sense as a generic term encompassing fats, lipids, alcohol-ethersoluble constitutents of protoplasm, which are insoluble in water. Lipids compose the fats, fatty oils, essential oils, waxes, steroid, sterols, phospholipids, glycolipids, sulpholipids, aminolipids, chromolipids, and fatty acids. The term encompasses both naturally occurring and synthetic lipids. Preferred lipids in connection with the present invention are: steroids and sterol, particularly cholesterol, phospholipids, including phosphatidyl and phosphatidylcholines and phosphatidylethanolamines, and sphingomyelins. Where there are fatty acids, they could be about 12-24 carbon chains in length, containing up to 6 double bonds, and linked to the backbone, the fatty acids could be different (asymmetric), or there may be only 1 fatty acid chain present, e.g., lysolecithins. Mixed formulations are also possible, particularly when the non-cationic lipids are derived from natural sources, such as lecithins (phosphatidylcholines) purified from egg yolk, bovine heart, brain, or liver, or soybean.

"Liposome" refers to a microscopic spherical membrane-enclosed vesicle (about 50-2000 nm diameter) made artificially in the laboratory. The term "liposome" encompasses any compartment enclosed by a lipid bilayer. Liposomes are also referred to as lipid vesicles. In order to form a liposome the lipid molecules comprise elongated nonpolar (hydrophobic) portions and polar (hydrophilic) portions. The hydrophobic and hydrophilic portions of the molecule are preferably positioned at two ends of an elongated molecular structure. When such lipids are dispersed in water they spontaneously form bilayer membranes referred to as lamellae. The lamellae are composed of two mono layer sheets of lipid molecules with their non-polar (hydrophobic) surfaces facing each other and their polar (hydrophilic) surfaces facing the aqueous medium. The membranes formed by the lipids enclose a portion of the aqueous phase in a manner similar to that of a cell membrane enclosing the contents of a cell. Thus, the bilayer of a liposome has similarities to a cell membrane without the protein components present in a cell membrane. As used in connection with the present invention, the term liposome includes multilamellar liposomes, which generally have a diameter in the range of about 1 to about 10 micrometers and are comprised of anywhere from two to hundreds of concentric lipid bilayers alternating with layers of an aqueous phase, and also includes unilamellar vesicles which are comprised of a single lipid bilayer. The latter can be produced by subjecting multilamellar liposomes to ultrasound, by extrusion under pressure through membranes having pores of defined size, or by high pressure homogenization. A further result of these procedures is, that often well defined size distributions of the liposomes are achieved. By extrusion through membranes of defined pore size (typical values are 100, 200, 400 or 800 nm), liposomes with a size distribution close to the pore size of the membrane can be achieved. By ultrasound and high pressure homogenisation procedures, defined size distributions are obtained by molecular self-organization as a function of the experimental conditions.

"Lysolipid" refers to a lipid where one fatty acid ester has been cleaved resulting in a glycerol backbone bearing one free hydroxyl group.

"Lysophospholipid" refers to a phospholipid where one fatty acid ester has been cleaved resulting in a glycerol backbone bearing one free hydroxyl group.

"Micelle" refers to an aggregate of amphiphilic molecules in a colloidal suspension.

"Nanoaggregate" refers to an assembly on a nanometer to micrometer scale assembled from smaller particles held together by attractive physical forces.

"Nanoparticle" refers to an entity in the dimensions of nanometers to micrometers.

"Negatively Charged Lipids" refer to lipids that have a negative net charge. In the present invention, it is referred to environments where the pH is in the range between 3 and 9, preferably between 5 and 8. Examples are phosphatidic acid, phosphatidylserine, phosphatidylglycerol, phosphatidylinositol (not limited to a specific sugar), fatty acids, sterols.

"Neutral Lipids" refer to lipids that have a neutral net charge such as cholesterol, 1,2-diacyl-sn-glycero-3-phosphoethanolamine, including but not limited to dioleoyl (DOPE), 1,2-diacyl-glycero-3-phosphocholines, Sphingomyelin. In the present invention, it is referred to environments where the pH is in the range between 3 and 9, preferably between 5 and 8.

"Particle diameter" refers to the size of a particle. To experimentally determine particle diameters, dynamic light scattering (DLS) measurements, using Malvern Zetasizer 1000 or 3000 (Malvern, Herrenberg, Germany) were performed. For quantitative data analysis (determination of Z (average and PI), in all cases parameters (refractive index, density, viscosity) of pure water were plugged in, even if the aqueous phase contained cryoprotectants to a certain extend. Therefore, for the absolute numbers, a certain systematic deviation with respect to literature data may have to be taken into account.

"Pegylated lipid" refers to a lipid bearing one ore more polyethylene glycol residues.

"Pharmaceutical composition" refers to a combination of two or more different materials with superior pharmaceutical properties than are possessed by either component.

"Phospholipid" refers to a lipid consisting of a glycerol backbone, a phosphate group and one or more fatty acids wich are bound to the glycerol backbone by ester bonds.

"Positively Charged Lipids" refer to a synonym for cationic lipids (for definition see definition of "cationic lipids"). In the present invention, it is referred to environments where the pH is in the range between 3 and 9, preferably between 5 and 8.

"Sterol" refers to a steroid alcohol. Steroids are derived from the compound called cyclopentanoperhydrophenanthrene. Well-known examples of sterols include cholesterol, lanosterol, and phytosterol.

"Therapeutic agent" refers to a species that reduces the extent of the pathology of a disease such as cancer. Such a compound may, for example, reduce primary tumor growth and, preferably, the metastatic potential of a cancer. Alternatively, such a compound may reduce tumor vascularity, for example either by decreasing microvessel size or number or by decreasing the blood vessel density ratio.

"Virtually free" of a species refers to as not detectable by HPTLC.

"Zeta potential" refers to a surface potential of a particle such as a colloidal particle measured with an instrument such as a Zetasizer 3000 using Laser Doppler micro-electrophoresis under the conditions specified. The zeta potential describes the potential at the boundary between bulk solution and the region of hydrodynamic shear or diffuse layer. For the present patent, zeta potential measurements were performed with a Malvern Zetasizer 3000 (Malvern, Herrenberg, Germany).

The present invention follows a strategy, which is contrary to the development of CPT drugs within the last 30 years, since it bases on using the carboxylate, form instead of the lactone from of a CPT drug. The inventive composition is obtained from CPT-carboxylate and a cationic organic counter molecule (Fig. 3), which is an amphiphilic low molecular weight compound, or a polymer. For the composition, the abbreviation CM-CPT (cationic molecule-CPT), will be used, independently if the organic counter molecule is a low molecular weight amphiphile or a polymer.

CM-CPT is characterized by its physico-chemical properties, which are clearly different to those of the constituting molecules. It can be identified as an independent molecular form in contrast to the carboxylate and the lactone form for example by spectroscopic measurements (Fig. 4, Fig.5). Contrary to the lactone form of CPT, which is lipophilic or the carboxylate form, which is hydrophilic, the properties of CM-CPT are determined by the organic counter molecule, i.e., it may be amphiphilic and it may be used, for the assembly of organized molecular nanoaggregates (Fig. 6). Unexpectedly, it turned out that the inventive composition is useful for producing a pharmaceutical preparation, since, it enhances the cytostatic activity of CPT while it overcomes the disadvantages of the carboxylate as well as those of the lactone form.

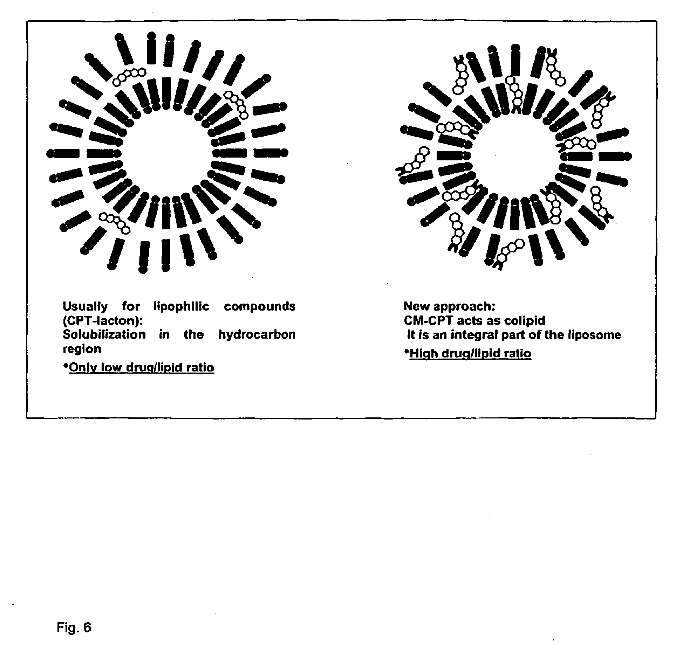

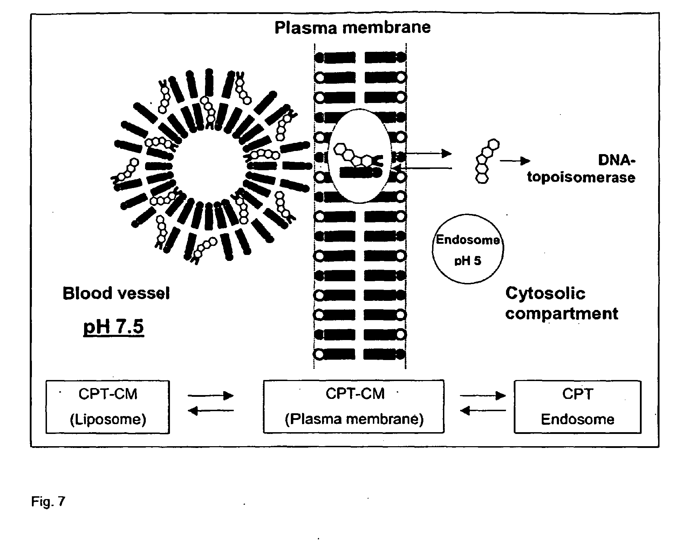

The advantages of the present invention are as follows:.
- With the inventive CPT-lipid composition, an active formulation is obtained, which does not have the disadvantages of the CPT drugs so far. The inherent difficulties of the lactone (low solubility and stability in aqueous environment) and the carboxylate form (low membrane permeability, low activity and induction of side reactions) are overcome. With CM-CPT, an active anti-cancer drug is provided, which does not show the side reactions as induced by the CPT-carboxylate.
- The composition can be brought into on aqueous environment and stable colloidal nanoaggregate can be formed. Liposomes with high drug/lipid ratios may be obtained (Fig. 6). The release of the free CPT-carboxylate and therefore side reactions are reduced.
- Due to the efficient binding to the cationic counter molecule and the embedding into a lipid matrix (such as a liposomal bilayer), CM-CPT is protected against blood/serum interactions and the deactivation of the CPT as induced by these interactions is suppressed.
- Since the ion pair of CM and the organic cationic contes molecule has amphiphilic properties, its membrane solubility is increased and cellular uptake of the drug is facilitated / enabled (Fig. 7). Different uptake mechanisms are possible. In addition to the so-called endocytotic pathway, fusion of liposome carrier with the cell membrane and concomitant direct release of the complex into the cytoplasma or direct permeation of the complex through the plasma membrane into the cytoplasm may occur.

It is well known, that, in on aqueous environment, amphiphiles as well as polymers mentioned herein can form various types of colloidal nanoparticulate systems. These include suspensions of micelles, liposomes, nanocapsules, any other type of nanoparticles or as well emulsions and gels. The physico-chemical properties of the described inventive composition are determined as well by the constituting amphiphile or polymer. Therefore, suspensions of stable nanoaggregate can be made from the inventive composition in an analogous way as with the constituting amphiphiles and/or polymers.

Thus, a further aspect of the present invention relates to a colloidal nanoaggregate comprising the inventive composition and having an overall positive charge. The proportion of a CPT drug in the cationic colloidal nanoaggregates is preferably less than about 50 mol% (in case of liposomes, the aqueous compartment is disregarded in this balance). In some embodiments, the nanoaggregate comprise a camptothecin drug in a proportion from about 0.1 mol% to about 50 mol% and in other embodiments from about 1 mol% to about 30 mol%. In other embodiments, a camptothecin drug is present in about 3 mol% to about 10 mol%.

The colloidal nanoaggregate may comprise cationic lipids in an amount of at least about 30 mol% to at most about less than 100 mol%, preferably 40 mol% to about 98 mol%, more preferably about 50 mol% to about 98 mol% in the surface layer.

The inventive nanoaggregate can further comprise amphiphiles with a negative and/or neutral net charge (anionic and/or neutral amphiphile). These can be selected from sterols or lipids such as cholesterol, phospholipids, lysolipids, lysophospholipids, sphingolipids or pegylated lipids with a negative or neutral net change. Useful anionic and neutral lipids thereby include: Phosphatidic acid, phosphatidylserine, phosphatidylglycerol, phosphatidylinositol (not limited to a specific sugar), fatty acids, sterols containing a carboxylic acid group, cholesterol, 1,2-diacyl-sn-glycero-3-phosphoethanolamine, including but not limited to dioleoyl (DOPE), 1, 2-diacyl-glycero-3-phosphocholines, sphingomyelin. The fatty acids linked to the glycerol backbone are not limited to a specific length or number of double bonds. Phospholipids may also have two different fatty acids.

In a preferred embodiment the neutral amphiphile is diacylphosphatidylcholine.

The inventive nanoaggregate may comprise neutral and/or anionic lipids in an amount of about 0 to about 70 mol%, preferably of about 20 mol% to about 50 mol% and most preferably of about 30 mol% to about 40 mol% in the surface layer.

In the context of the present invention overall positive charge means an excess of positively charged molecules in the molecular surface layer of the colloidal nanoaggregate. Thus, in a preferred embodiment, the inventive nanoaggregate comprises an excess of cationic lipids of at least 20 mol%, preferably at least 30 mol% and most preferably at least 40 mol%. As an example, if the surface of the nanoaggregate contains 10 mol% negatively charged lipids or negatively charged CPT drug, the amount of positively charged lipid has to be at least 30 %, preferably at least 40 mol% and most preferably at least 50 mol% in order to fulfil these charge requirements. Measurements of the zeta potential can be used to proof the net charge of the colloidal nanoaggregates. Preferably, zeta potentials are in the range between 25 mV and 100 mV, more preferably between 35 mV and 70 mV, depending on the composition (measured in about 0.05 M KCl solution at about pH 7.5 at room temperature are useful in the context of the present patent. However, measured under these conditions, zeta potential values lower than 25 mV can be determined for polymer graft colloidal nanoaggregates, for example when the surface of the nanoaggregates comprises pegylated lipids.

The inventive nanoaggregates can be present as different types of supramolecular assemblies with different size ranges. Thus, in further preferred embodiments, the inventive nanoaggregate is an emulsion droplet, a liposome, a micelle, a nanoparticle or a nanocapsule. The particle diameter may range from about 5 nm to about 2000 nm, preferably between 50 nm and 1000 nm and more preferably between 100 to 500 nm.

Preferred nanoaggregates of the present invention are cationic liposomes comprising DOTAP, DSTAP, DPTAP, DMTAP, DLTAP, DODAP, other analogues of DOTAP or DODAP or any other cationic lipid in an amount, that the net excess of positively charged moieties is at least about 20%, preferably of at least about 30mol%, more preferably of at least about 40%, and most preferably of at least about 50% with respect to the total number of molecules. The liposomes may further contain DOPC, pegylated lipids or any other lipid that has a neutral or negative net charge. The fraction of pegylated lipids is preferably up to about 10%, more preferably up to about 5%. In case the liposomes do not contain pegylated lipids, the zeta potential of this liposomes is preferably above about 30 mV, more preferably above about 40 mV, and most preferably above about 50 mV. If pegylated lipids are present or any other lipid, the zeta potential of the preferred formulation as measured under the conditions given above can be as well be lower, i.e. in the range close to zero.

Within the present invention an inventive cationic colloidal nanoaggregate such as a liposome, can be freeze-dried (lyophilized), stored for an extended time period and then reconstituted by adding the removed amount of water to the dry lyophilisates, Reconstitution of the lyophilisates is performed when and where they are to be used without losing a substantial portion of their contents during the freeze-drying process (lyophilization), storage and reconstitution step. To achieve the latter, one or more protective agents such as cryoprotectants can be present. Thus, preferably the inventive nanoaggregate comprises a cryoprotectant wherein the cryoprotectant is selected from a sugar or an alcohol or a combination thereof. Preferably, the cryoprotectant is selected from trehalose, maltose, sucrose, glucose, lactose, dextran, mannitol or sorbitol. The cryoprotectant can be present in an amount of about 1 % to about 20%, preferably 5% to 15%, most preferably 6% to 12%, with respect to the aqueous solution (m/v) from which the lyophilisate is formed. The corresponding molar ratio lipid/cryoprotectant depends on the liposome concentration. For a 30 mM formulation, the molar ratio is about 1:1 to 1:20, preferably 1:5 to 1:15, most preferably 1:6 to 1:12. For the molar ratios for other liposome concentrations these numbers have to be multiplied by the factor as given by the relation between the two concentrations.

Also disclosed is a concentrated lipid and CM-CPT drug suspension or gel. The concentration of the suspension or gel is preferably higher than 100 mM, but it can be as well at 200 mM, 500 mM or higher. The concentrate may be stored as it is, or freezing or lyophilization may be applied. One or more protective agents such as cryoprotectants, buffers or other additives can be present. The concentrate can be reconstituted with an aqueous phase, which can contain buffers, and other additives in order to obtain a more diluted liposome suspension.

In accordance with other aspects of the invention, a pharmaceutical preparation comprising a pharmaceutically effective amount of any claim 16 together with a pharmaceutically acceptable carrier, diluent and/or adjuvant is provided.

A further aspect of the present invention relates to a method of producing the inventive nanoaggreagates. Colloidal nanoaggregates can be formed by various methodologies, well known to ordinarily skilled artisans. Sonication, vortexing, mechanical stirring, static mixing, homogenization, injection, microfluidization, colloid mills and/or pressure emulsifiers can be used to prepare nanoaggregates of various types including various orders of addition of materials. The nanoaggregates of the present invention can be formed by a homogenization, a lipid film, a solvent mixing or by a solvent injection procedure.

The nanoaggregates of the present invention can be produce by a method comprising the following steps:
a) providing a camptothecin drug, preferably as a camptothecin carboxylate,
b) contacting said drug with a cationic amphiphile which has a positive net charge (cationic amphiphile) under conditions wherein said drug associates in its carboxylate form with said cationic amphiphile,
c) optionally admixing at least one further amphiphile in step b) which has a negative and/or neutral net charge (anionic and/or neutral amphiphile) and
d) forming a colloidal nanoaggregate with an overall cationic charge as described above.

Preferred procedures for preparing CPT-carboxylate are as follows:
i) the lactone is dissolved in a suitable alkaline environment, and then, optionally, the solution is brought to the desired pH or
ii) the lactone is dissolved in a suitable alkaline environment, and dry carboxylate salt is obtained by drying, recristallization or precipitation or
iii) the lactone is dissolved in a volatile alkaline solvent (e. g. ammonia in a suitable concentration) and the solvent is subsequently evaporated. Following, the pure CPT-ammonium salt, without other additives is obtained.

In each case standard inorganic or organic bases can be used, such as NaOH, KOH, NH₄OH TRIS, etc. The solution can comprise H₂O and organic solvents (methanol, ethanol, propanol, isopropanol, acetone) in any suitable relation.

The solvent injection procedure is performed wherein
a) an organic solution comprising at least one cationic amphiphile and optionally at least one anionic and/or neutral amphiphile is added to an aqueous solution of the carboxylate form of a camptothecin drug or a derivative thereof or
b) an organic solution comprising at least one cationic amphiphile and optionally at least one more cationic, anionic and/or neutral amphiphile and the camptothecin drug or a derivative thereof is added to an aqueous solution,
wherein said aqueous solution has a pH value between 3 and 9, preferably between 5 and 8.

The solvent mixing procedure is performed wherein an aqueous solution and a non-aqueous solution are mixed, where the solutions can contain cationic amphiphiles, anionic amphiphiles neutral amphiphiles, polymers buffers, cryoprotectants and other additives, and at least part of the amphiphiles are dissolved in the non-aqueous phase. Then the non-aqueous solvent and other undesired components are removed by suitable methods, preferably evaporation or dialysis. A colloidal suspension of liposomes or a concentrated gel, comprising lipids and a camptothecin drug is obtained. The gel or suspension can be frozen or lyophilised for storage. It can be reconstituted with suitable aqueous phase to give the desired colloidal nanoaggregates.

Alternatively, in a preferred embodiment a lipid film can be formed by two different methods:
The camptothecin drug can be incorporated either directly by dissolving it in an organic solution comprising at least one cationic amphiphile and optionally at least one anionic and/or neutral amphiphile and the camptothecin drug or by forming a lipid film without the drug first. In the first case, the lipid film is subsequently taken up by an aqueous solution comprising no drug and in the second case with the camptothecin drug.

In a further preferred embodiment, the organic solution comprises an organic solvent selected from methanol, ethanol, propanol, isopropanol, ethylene glycol, tetrahydrofuran, chloroform or diethylether or a mixture of these solvents.

The aqueous solution may further comprise a cryoprotectant, which is selected from a sugar or an alcohol or a combination thereof such as trehalose, maltose, sucrose, glucose, lactose, dextran, mannitol or sorbitol in the range of up to about 20 % (m/v). Preferably the stabilizing agent is used in the range of about 1 % (m/v) to about 20 % (m/v) and most preferably in the range of about 5 % (m/v) to about 15 % (m/v) with respect of the total volume of the liposomal dispersion. The corresponding molar ratio lipid/cryoprotectant depends on the liposome concentration. For a 30 mM formulation, the molar ratio is preferably about 1:1 to 1:20, most preferably 1:5 to 1:15. The molar ratios for other liposome concentrations are given from these numbers multiplication with the factor as given from the relation of the two concentrations.

In an other preferred embodiment the inventive method can further comprise at least one dialysis and/or at least one homogenization and/or optionally at least one sterile filtration and/or optionally a freeze-drying and/or optionally a reconstitution step. Dialysis might be necessary in order to remove free camptothecin drug, or in order to remove non-aqueous solvent or other components from the solution. Since the nanoaggregates as achieved by the above-described method do not necessarily have the desired size distribution, a homogenization step may be performed subsequently. Homogenization can be performed by extrusion through a membrane with defined pore size, high-pressure homogenization, ultrasound treatment, high speed homogenization, or any other homogenization technique well known in the art.

In another embodiment of the inventive method an aqueous solution of the camptothecin drug is added to a suspension of preformed cationic liposomes without active compound. The liposome suspension can be at a concentration ranging from 5 mM to 100 mM lipid or it can be at concentrations from 100 mM to 500 mM lipid or higher (gel).

Angiogenesis associated diseases are dependent on blood supply. The local interruption of the vasculature will produce an avalanche of cell death. The vascular endothelium is in direct contact with the blood. It is contemplated that a variety of diseases can be prevented and treated with the foregoing methods and compositions.

Thus in another aspect, a composition, a colloidal nanoaggregate or a pharmaceutical formulation as provided by the present invention is used for producing a medicament for preventing and/or treating a disease such as cancer, a variety of inflammatory diseases, diabetic retinopathy, rheumatoid arthritis, inflammation, dermatitis, psoriasis, stomach ulcers, macular degeneration, hematogenous and solid tumors. Further, the methods and compositions of the present invention can be applied for producing a medicament for preventing and/or treating solid tumors and their metastases such as bladder, brain, breast, cervical, colorectal, endometrial, head and neck or kidney cancer, leukemia, liver or lung cancer, lymphoma, melanoma, non-small-cell lung, ovarian, pancreatic or prostate cancer.

The compositions and nanoaggregates of the invention are particularly suitable for the targeting of endothelial cells. Thus, they can be used for producing a medicament for the selective delivery of a camptothecin drug with antimitotic activity to an angiogenic vascular target site as defined above, wherein delivery comprises the steps of
a) providing a membrane permeating composition of a camptothecin drug or a derivative thereof, and/or a colloidal nanoaggregate and/or a pharmaceutical composition of the invention,
b) administering said composition and/or colloidal nanoaggregate and/or pharmaceutical composition to a subject in need thereof,
c) allowing the composition and/or colloidal nanoaggregate and or pharmaceutical composition to associate with the target site and
d) releasing and thereby activating said camptothecin drug or said derivative at the target site.

In a preferred embodiment, in step a) an inventive composition and/or an inventive colloidal nanoaggregate and/or a pharmaceutical composition thereof is provided.

Preferably, step d) comprises endocytosis and/or fusion with the cell membrane and/or passive or active diffusion across the cell membrane,
wherein endocytosis comprises releasing said camptothecin into the lysosomal compartment.

### Figure legends

- Fig. 1:: Equilibrium between camptothecin in the lactone form (left) and the carboxylate form (right). The equilibrium is pH dependent and reversible. At pH values close to 7.5 and above, the carboxylate form predominates.
- Fig 2:: A reason for the enhanced cytotoxicity of the CPT-carboxylate may be its reduced membrane permeability due to the negative charge. Therefore the delivery to the target site is inhibited. On the other hand, in organs, where a more acidic pH is present, the CPT may reorganize into the lactone form and then it may penetrate more easily across the cell membrane, thus inducing the toxic side effects.
- Fig. 3:: Equilibria between the carboxylate form, the lactone form and the cationic molecule-bound form of CPT (CM-CPT).
- Fig. 4:: Excitation and fluorescence spectra of camptothecin, dissolved in chloroform/methanol (free CPT) and CM-CPT as a liposomal formulation in aqueous environment at pH 7.5 (Liposomal CPT).
- Fig. 5:: UV-vis spectra of CPT under different conditions. The spectrum of the liposomal CPT (top curve) differs clearly from those under all other conditions. Characteristic shapes can be made out for the liposomal CPT, the CPT-carboxylate (second and third curve from above) and the lactone from of the CPT (fourth and fifth curve from above). The lactone spectrum shows as well a strong dependence on the solvent environment. For clarity the spectra are vertically shifted.
- Fig. 6:: Liposomal organization of CPT in the lactone form (left) and in the carboxylate form associated with a cationic amphiphile (right). Only a limited amount of the lactone can be embedded in the hydrophobic compartment of the liposome. Due to the amphiphilic properties of CM-CPT, it can act as an integral part of the membrane and much higher drug/lipid ratios can be obtained.
- Fig. 7:: Cellular uptake via the plasma membrane. The association of CPT with the amphiphile enhances the lipophilic character. Therefore, processes as insertion into the plasma membrane after binding and/or fusion, and permeation across the membrane can be facilitated. The pH shift on endocytosis favours the release of CPT in the lactone form.
- Fig. 8:: Dialysis of lipid suspensions together with CPT-carboxylate present in the solution. The solutions were 10 mM in lipid, 1 mM CPT-carboxylate, pH 7.5. With DOPC virtually all CPT is released into the dialysate, while with DOTAP a substantial retention effect is determined.
- Fig. 9:: Fluorescence anisotropy measurements of CPT in lipid suspensions from DOTAP and DOPC and as prepared by different methods. The measurements were performed with 10 mM Tris-buffer, pH, 7.5 and with 10 % trehalose present in the aqueous phase. For DOPC the standard film method was used (second column). The third column shows the results for liposome formation from DOTAP and CPT in the lacton form by the film method and the third column shows the results when CPT-carboxylate was used instead of the lactone. The fifth column shows ethanol injection into aqueous solution of CPT-carboxylate. Only with DOTAP a substantial anisotropy was detected. The value was highest with the ethanol injection procedure, indicating that in this case as well the binding efficacy was highest.
- Fig. 10:: Comparison of fluorescence anisotropy measurements with DOTAP and DMTAP Measurements with CPT and with 10-OH-CPT are shown. For DMTAP, measurements at RT and at 40°C were performed. DMTAP, having saturated fatty acid chains, is present in a fluid-like state at 40°C and in a solid-like state at RT. DOTAP is in a fluid like state already at RT. With DMTAP in the fluid-like state, the anisotropy is as high, of higher, as with DOTAP. With 10-OH-CPT the anisotropy is even higher as with CPT.

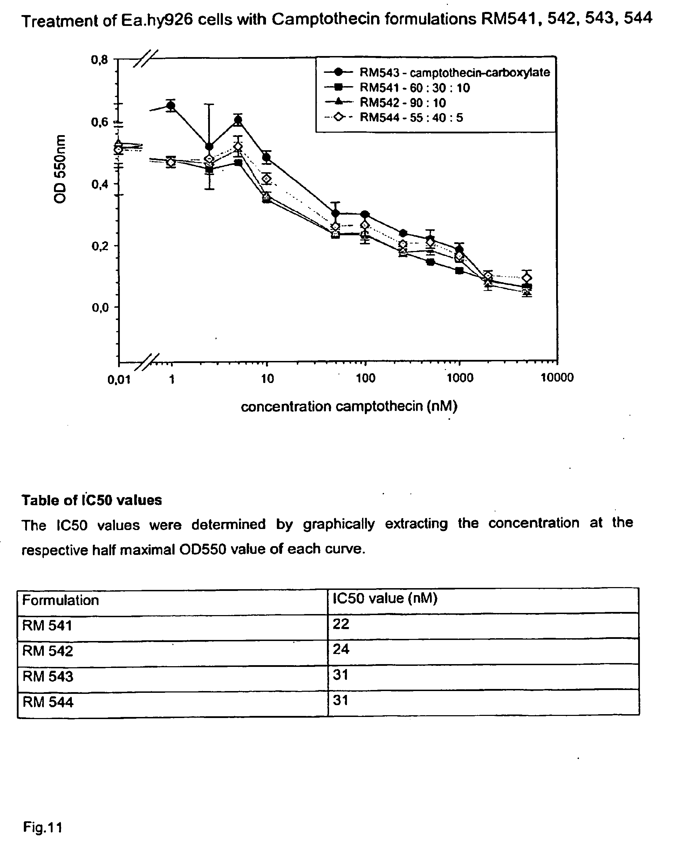

- Fig. 11:: Inhibition of Ea.Hy 926 cell growth by Camptothecin formulations RM541, 542, 543, 544. For details see text.

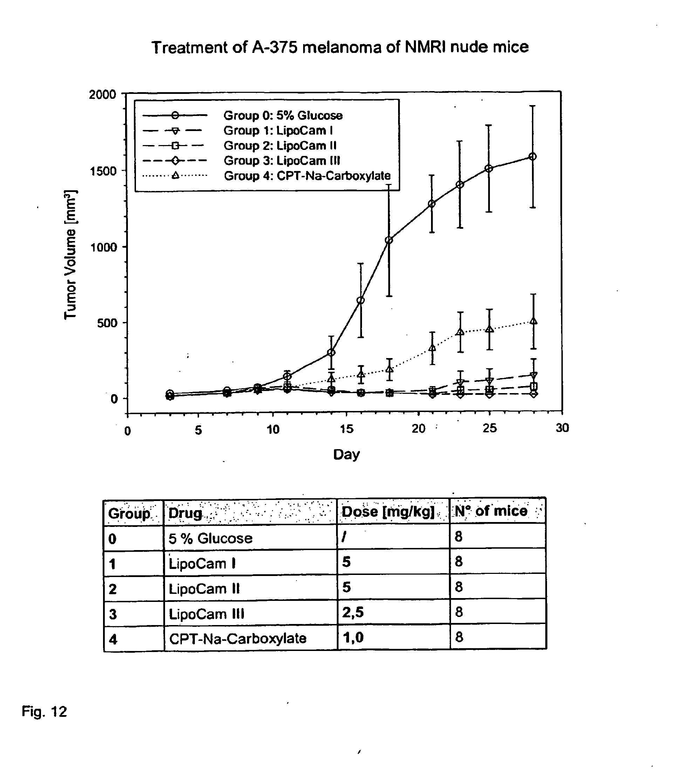

- Fig. 12:: Therapeutic efficacy of LipoCam in A-375 melanoma of nude mice. For details see text.

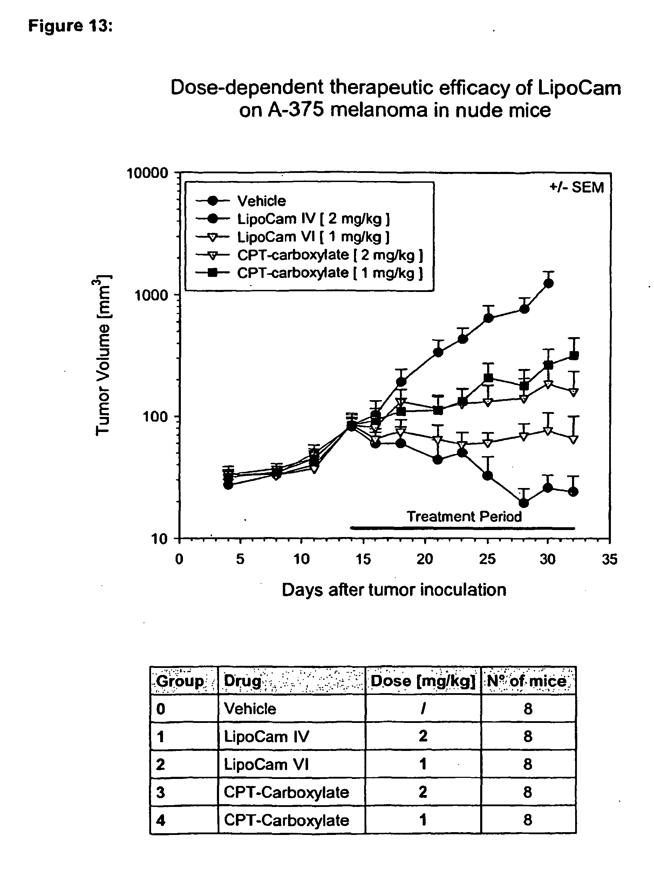

- Fig. 13:: Therapeutic efficacy of LipoCam IV and VI in A-375 melanoma of nude mice. For details see text

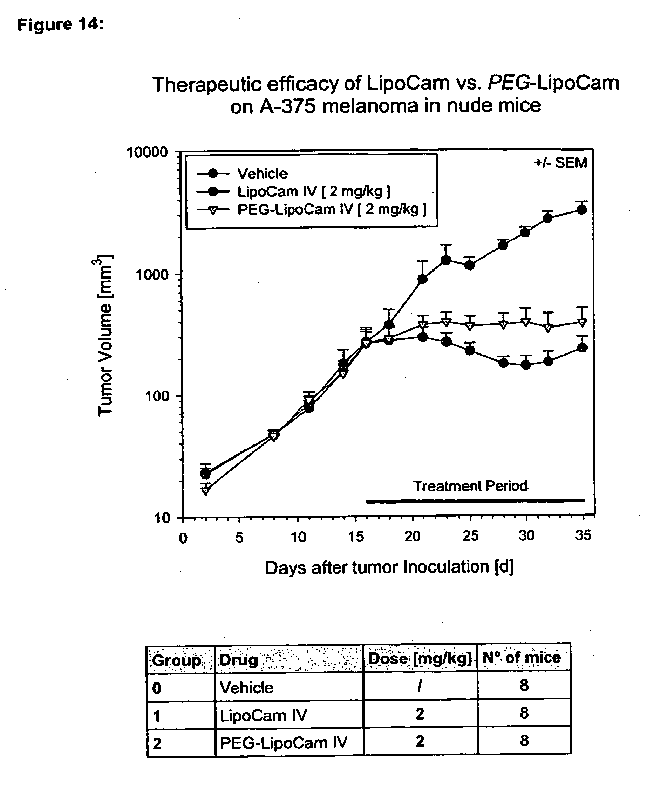

- Fig. 14:: Therapeutic efficacy of LipoCam IV and PEG-LipoCam IV in A-375 melanoma of nude mice. For details see text

The following examples should be illustrative only but are not meant to be limiting to the scope of the invention. Other generic and specific configurations will be apparent to those skilled in the art.

### Examples

### 1. CPT-carboxylate salt preparation

The carboxylate salt of a CPT drug is obtained by exposing the CPT drug to an alkaline environment, preferably with a pH above 9. Explicitly, two procedures comprising the following steps, were applied:
a) The CPT drug is dissolved in of NaOH, i.e., the molar ratio NaOH/CPT has to be ≥ 1. Already a molar ratio of 1:1 is suitable to dissolve the CPT, but as well higher excess of the alkaline compound can be used. The excess can be as small as 1.05, i.e., for a 100 ml of a CPT-carboxylate solution which has a drug concentration of 100 mM, 10 mMoles of CPT are dissolved in 100 ml 1.05 mM NaOH. Concentrations as high as 20 mM can be achieved. The solution may contain as well other additives, such as ions or cryoprotectants. Subsequently, if necessary, by adding buffers or acids (Bis, Tris, HEPES, HCl), the solution can be brought to a lower or higher pH if desired. The solution can be diluted, concentrated (for example by solvent evaporation) and further components (ions, buffers) can be added. The Na-CPT salt can be precipitated an obtained as a dry pure compound, if desired. Another suitable way to produce the dry CPT carboxylate is lyophilization. For the procedure as well any other base or suitable alkaline compound can be applied (KOH, NH₄OH Tris, etc.).
b) The drug is dissolved in excess of a volatile base, and then the base is evaporated and the pure CPT carboxylate salt is obtained. For example, 2 mg/ml of CPT drug can be dissolved in 25% NH₄OH. Subsequently the solvent is evaporated. In this way, the pure ammonium salt of the CPT drug-carboxylate is obtained. For further treatment, it can be brought in aqueous phase of a desired pH and selected environmental conditions (buffers, cryoprotectants). Alternatively it can be brought into a suitable organic phase.

### 2 Synthesis of a CM-CPT composition

A CM-CPT (composition of cationic molecule and camptothecin) is obtained from CPT-carboxylate salt by adding a solution of a cationic amphiphile to the carboxylate salt. If desired, subsequently the solvent can be evaporated. The solvent can be Ethanol, Chloroform, Chloroform/Methanol, or any other suitable organic solvent or solvent mixture. Alternatively, the solution of the cationic amphiphile can be added to an aqueous solution of the CPT-carboxylate. In case of a positively charged polymer as a countermolecule, the latter can be present as well in the aqueous phase. For further treatment, the CM-CPT solution or suspension can be used as it was prepared, or the solvent can be removed in order to obtain the pure composition. Subsequently it can be brought into a desired medium.
For example in order to obtain a 1 mM CPT-DOTAP composition with a molar ratio of about 1:1, 1 mmol of CPT is dissolved in NH₄OH and then the solvent is evaporated according to procedure 1 b). To the remaining salt (in that case present as a thin film at the inner wall of a flask), 1000 ml of a 1 mM solution of DOTAP in EtOH are added. In other cases, if this is favourable for further formulation, lower molar ratios CM/CPT may be realized, such as, 10/90, 5/95, 1/99 or 0.5/99.5.

### 3. Liposome formulation

The formulation procedure may consist of the following steps:
1. Providing CPT carboxylate
2. (optional) Providing CM-CPT
3. Formation of a colloidal suspension from the lipid and drug components by a suitable method
4. (optional) Extrusion through a membrane in order to achieve unilamellar liposomes of uniform size
5. (optional) Dialysis or analogous methods for the separation of non-encapsulated drug and/or components or solvent
6. (optional) A concentration step to achieve a highly concentrated liposomal gel
7. (optionally) Freeze drying
8. (optionally) Freezing
9. (optionally) Reconstitution under suitable environmental conditions

For the formation of the colloidal liposome suspensions, different methodologies can be applied. Subsequently, the most relevant techniques are briefly outlined.

### Film method

Liposomes can be formed by the well-known film method, such as described in the literature. Briefly, from the organic solution of the lipid, or the lipid plus the drug, the solvent is evaporated, and a thin film of lipid (or lipid plus drug) is formed at the inner wall of a flask. The thin molecular film is resuspended in an aqueous phase, which can contain further components such as buffers, ions, cryoprotectants and the like. With this procedure, liposomal suspensions are formed in a self-assembly process. A standard formulation is obtained by forming a film of 90 µmol DOTAP and 10 µmol camptothecin from a solution in chloroform/methanol, 10/1. The film is then reconstituted with 10 ml of the aqueous phase, in order to achieve a suspension where the total liposomal concentration (lipid + drug) is 10 mM.

The aqueous solution can contain a cryoprotectant, e. g. glucose or trehalose and/or buffers (Tris), to achieve a desired pH after reconstitution. A typical pH is in the range between 7 and 8. However, depending on the lipid, as well higher (up to pH 9) or lower (down to pH 5) values can be selected. For the cryoprotectant, typical conditions correspond to 5-10% of trehalose present in the aqueous solution. The pH of the liposomal formulation can be altered after formulation for further treatment.

Thus, a liposomal formulation with the drug/lipid ratio of 1:9, and with a total (lipid+drug) concentration of 10 mM is obtained. Other typical molarities are 15 mM, 20 mM, 25 mM or 30 mM. If necessary, molarities up to 50 mM or higher can be formulated. In that cases, the same formulation procedure as described for 10 mM is applied, where the total amounts of drug, lipid and solvent are adopted to the desired concentration. The drug:lipid ratio usually is selected to be in the rage from 1:99 to 20:80. Another frequently used molar ratio is 5/95. These formulations can be realized by the above described procedure with the molar amounts of the components adopted to the desired final formulation.

For example, for 10 ml of a 25 mM formulation with a molar composition of 5/95, a film of 23.75 µmol DOTAP and 12.5 µmol camptothecin is reconstituted with 10 ml of buffer. The lipid phase can consist of only cationic lipid, such as DMTAP, DOTAP, DPTAP, DSTAP, DOTMA, or DDAB, or it can comprise up to 60% of charged and non-charged colipids.

Standard formulations which have been used by us most frequently consist of CPT/DOTAP/DPOC = 5:47.5:47.5, or 5:55:45 or 10:45:45 or 10:60:30.

In table 1 the results form physico-chemical characterization of such liposomal formulations are given. The listed formulations have been tested as well in an efficacy study (Fig. 12) Accordingly to the described procedure, formulations comprising other cationic lipids, such as DMTAP, DSTAP, DDAB, DOTMA and the like can be achieved.

**Tab. 1: Results from physico-chemical characterization of frequently used liposomal formulations**

| Formulation | Composition DOTAP/DOPC/CPT | Total molarity [mM] | Size [nm] | PI | Zeta potential (mV) |
|---|---|---|---|---|---|
| | | | | | |
| Lipocam I | 55/40/5 | 10 | 189.4 | 0.116 | 54 |
| | | 15 | | | |
| | | 25 | | | |
| Lipocam II | 60/30/10 | 10 | 179 | 0.146 | 57 |
| | | 15 | | | |
| | | 25 | | | |
| Lipocam III | 90/10 | 10 | 194 | 0.131 | 65 |
| | | 15 | | | |
| | | 25 | | | |

In an alternative embodiment of procedure 1a) the camptothecin is converted into the carboxylate, or the carboxylate salt for formulation. Such a way the CPT is 'activated' for easier association of the drug to the cationic lipid, as, in fact, the carboxylate is the molecular form which associates to the latter. CPT is solubilized in NH₃, and the volume which contains the necessary amount of the drug (i.e., 10 µM in the example described in 1 a) is added to a flask. After evaporation of the solvent a film of the ammonium salt of the CPT-carboxylate is formed. Then the organic solution of the lipid is added and the solvent is evaporated. Thus a film comprising camptothecin and lipid is obtained, where the camptothecin binds more readily to the cationic lipid. Particularly for low pH values, it is easier to obtain liposomes with high drug/lipid values. The subsequent procedure is analogous to the one described above.

In a further embodiment, the CPT-carboxylate can be part of the aqueous solution for reconstitution of the pure lipid film. In that case, a separation step (such as outlined below), or a concentration step (such as below) can be performed after formulation.

### Organic Solution Injection

Liposomal suspensions can be achieved through the injection of a solution of the lipid, or the lipid +drug, into the aqueous solution. A typical solvent for the lipid or the lipid+drug phase is ethanol ('ethanol injection'). In the present case, a new procedure is employed, in the sense that ethanol injection into the aqueous solution of the camptothecin-carboxylate is performed. As due to the attractive interaction between the drug and the cationic lipid, camptothecin-containing liposomes are formed in a self-assembly process. In order to achieve 10 ml of a 10 mM suspension of CPT/DOTAP 1:9, a 10 µM solution of CPT-carboxylate, optionally containing further additives as outlined in the previous section, is prepared. Other typical molarities are 15 mM, 20 mM, 25 mM or 30 mM of the total lipid +drug concentration. In order to obtain 10 ml of a 25 mM suspension of CPT/DOTAP 5:95, a 12.5 µM solution of CPT-carboxylate, optionally containing further additives as outlined in the previous section, is prepared. The solution of the carboxylate can be prepared directly by dissolving CPT-lactone in a suitable amount of base, or it can be prepared from dry salt. Preferentially, the ammonium salt solution or the Na-salt solution of the CPT-carboxylate is used.

The aqueous phase can have a pH between 5 and 9, according to the necessities of the experiment. In the examples which are presented here (tab. 2) a pH of 7.5 was selected. The ethanolic lipid solution has a concentration between 200-400 mM. The suitable volume of the lipid solution in ethanol is injected under vigorous stirring. All compositions and concentrations as described in the previous section can be achieved by this approach. In case of the 10 mM 1:9 suspension, ca. 0.23 ml of a 400 mM DOTAP solution in Ethanol can be used and in case of the 25 mM 5:95 suspension, ca. 0.59 ml of a 400 mM DOTAP solution in Ethanol can be used. As an alternative to ethanol, as well other suitable solvents or mixtures thereof can be taken. Typically, these are alcohols, ethers, chloroform, hydrocarbons, etc. As well solvents in the supercritical state can be applied, such as hydrocarbons, carbon dioxide, perfluorinated compounds, etc. Subsequently to the described formulation procedure, extrusion (2) dialysis (3), a concentration step (4) or freeze drying can be performed.

Alternatively to the injection of the pure lipid solution, both, the lipid(s) and the CM-CPT drug can be dissolved in the injection solution. All other steps are equivalent. In the described formulation procedures, for better perceivability DOTAP and CPT were taken as representatives, however, the procedures are applicable for the other listed lipids and CPT drugs. Examples for formulations which have been performed with the described procedures with DMTAP, DSTAP, and DDAP as cationic lipids are given in Tab. 4 (Appendix). As well formulations with several colipids and pegylated lipids (DOPC, Cholesterol, PEG-DOPE) are listed. Depending on the used cationic lipid, the fraction of the colipid can be from 1 to 70 mole %. Pegylated lipids are preferentially used in the range from 1 to 15 mole %.

Further, in the table formulation examples with other CPT drug are given. Formulations with other CPT derivates are formed in the analogous way. In order to optimise the results, the explicit formulation procedure is adopted according to the molecular properties of the CPT drug and the cationic lipid. In this context the CPT drugs which are (i) more hydrophilic, which are (ii) more hydrophobic or (iii) which have similar properties as CPT can be distinguished. For CPT drugs which are more hydrophilic, preferentially ethanol injection into the aqueous solution of the drug, as described above, is performed. Examples for such molecules are Topotecan and Irinotecan, and formulation examples with these compounds are listed in Tab. 4. For CPT drugs which are more hydrophobic than CPT, preferentially film method, or ethanol injection where both, drug and lipid, are present in the ethanol solution, is performed. An example for a more hydrophobic derivate is CPT-oleate, which can be obtained from 10-OH-CPT as described in the literature (Lundberg 1998). A formulation example with CPT-oleate using the film method is given in Tab.4. Examples for compounds which, in the present context, have similar molecular properties as CPT are 10-OH-CPT and SN38. Therefore, as for CPT, either of the described methods can be applied. In Tab. 4 formulation examples with 10-OH-CPT and SN38 using ethanol injection are given.

### Extrusion

The liposomal suspensions as achieved by the above-described methods do not have necessarily the desired size distribution. Therefore, an extrusion through a membrane of defined pore size can be performed subsequently.

In our experiments we have usually performed extrusion through membranes of 200 nm pores size (Osmonics Inc., Poretics, polycarbonate 0.2 Microns). Other typical extrusion membranes were with 100 nm or with 400 nm pore size. Size distributions were controlled by quasi-elastic light scattering (Malvern, Herrenberg, Germany). Examples of liposomal sizes after extrusion are given in Tables 1 and 2.

### Separation of low molecular compounds

Dialysis techniques were used to separate low molecular components from the liposomal suspensions. This was particularly the case for the experiments with ethanol injection, where in addition to a fraction of non-liposomal camptothecin, a certain amount of ethanol was present in the suspension. Dialysis tubes from regenerated cellulose with a MWCO of 8,000-10,000 (Roth, Karsruhe Germany) were used. Alternatively, the so called "cross-flow" technique (Vivascience, Sartorius Group, Göttingen, Germany) with Polyethersulfone membranes, MWCO 50,000 were used. The release of the free CPT was monitored by UV-vis spectroscopy. The amount of the released CPT-carboxylate depends on the lipid and drug concentration, the drug/lipid ratio, pH and other parameters. An example is given in Fig.8, where the results from dialysis of 10 mM liposomes suspensions DOTAP/CPT and DOPC/CPT 90/10 are shown. With a dilution factor of 20, more that 60% of the CPT was retained by the DOTAP liposomes, while with DOPC liposomes, less that 10 % were retained, i.e., virtually all CPT was released.

### Freezing and Freeze-drying

Freeze drying of the liposomal formulations was performed using standard equipment and adopted protocols (for example: Epsilon 2-12D, Control Unit LMC-2, Christ, Osterode, Germany). The composition and the physical state of the suspensions were characterized after reconstitution such as described below. The suspension as described above could be frozen and brought back to room temperature, without drastically affecting the aggregation state of the liposomes, such as proven by quasi-elastic light scattering measurements.

Results from size measurements before and after lyophilization/freezing are given in Tab. 2.

Freeze drying enables to realize formulations which in liquid formulations are unstable. For example, thermodynamically metastable states can be 'frozen' and, after reconstitution, such formulations can be obtained with a reasonable in-use stability. Formulations, which in the liquid state have only a stability of hours or days can be frozen and reconstituted directly before application. Therefore, freeze drying permits to apply compositions, which have a stability of only few days or hours. In the context of the present invention, freeze drying permits to realize formulations in a wider pH range as it is possible in liquid formulations. In liquid formulations with CPT and DOTAP, the preferential pH range is between 6.5 and 8. Below, CPT-lactone formation is favoured and above lipid hydrolysis may occur. For lyophilisates as well formulations with lower (down to 4) and higher (up to 8-9) pH values can be realized.

Further, by using volatile acids or bases, favourable pH conditions during formulation and after lyophilization can be achieved, and these conditions can be selected different for the state during formulation, i.e., before lyophilization, and after lyophilization, i.e., for the reconstituted lyophilisates. By adding HCl during formulation, the pH can be lowered during that production state. As the HCl is lost on lyophilization, the pH of the suspension after reconstitution is again higher. This enables to increase in-use stability of compositions from a CPT drug and a cationic molecule,
where the lactone-carboxylate equilibrium of the CPT drug is shifted towards the lactone and the binding constant to the cationic molecule is low. By using NH₄OH during formulation, a camptothecin drug can be 'activated' for efficient binding to the cationic molecule (higher pH is favourable for carboxylate formation) before lyophilization. After lyophilization the excess NH₄OH is lost, and the thus obtained lower pH can be favourable for lipid stability.

**Tab. 2: Results from size measurements of selected liposomal suspensions of DOTAP comprising CPT. Measurements after extrusion (200 nm pore size), after diafiltration (to remove free CPT-carboxylate) and after lyophilization, storage and reconstitution to the original concentration are given. The results demonstrate, that, under suitable conditions, the suspensions can be lyophilised for storage and reconstituted without profoundly affecting the physical state of the liposomes.**

| Formulation | Extrusion Zₐᵥₑ [nm] PI | Diafiiltration Zₐᵥₑ [nm] PI | Lyophilization Zₐᵥₑ [nm] PI |
|---|---|---|---|
| RM622 | 196 | 195 | 193 |
| DOTAP/CPT 90110 | 0.082 | 0.096 | 0.112 |
| 10 mM | | | |
| RM623 | 191 | 204 | 203 |
| DOTAP/CPT 90/10 | 0.179 | 0.207 | 0.2 |
| 15 mM | | | |
| RM624 | 207 | 211 | 258 |
| DOTAP/CPT 90/10 | 0.409 | 0.4 | 0.545 |
| 20 mM | | | |

### 4. Concentrated liposome suspensions and gels

Pre-formed liposome suspensions are concentrated by standard techniques such as dialysis against polymer solution, 'cross-flow', or evaporation of solvent. The concentrated suspensions can be brought back to the previous, or any other concentration, without substantial changes of the original size distribution.

To directly prepare concentrated liposome suspensions and gels, an aqueous phase, containing (optionally) one or more of the components, and an organic phase, containing the other components are mixed. For example, an aqueous solution of 2 mM CPT carboxylate, 0.5% trehalose and 1 % tris/HCl buffer is mixed with a 6 mM solution of DOTAP in ethanol, that the final CPT-carboxylate/DOTAP ratio is 10/90. A homogeneous mixture is obtained. The solvent is then evaporated under reduced pressure at room temperature, until the desired concentration is obtained. It is possible to evaporate only the ethanol (together with the eutectic fraction of water) or, with higher vacuum, as well the water. It is possible to add ethanol to the semi-concentrated suspension for further evaporation. In this way, for example, suspension with a concentration of 100 mM, 200 mM and 500 mM can be obtained. After reconstitution with water or buffer to a lower concentration, a liposome suspension, where the size distribution is a function of the previous maximum concentration is obtained. Examples are given in Tab. 3.

In a further realization of this concept, concentrated gels of pure lipid are prepared for storage. Before application, the gels are diluted with an aqueous solution of a CPT drug. In this way, liposomal formulations of the CPT-CM compound can be achieved. Storage problems of the formulation are avoided, because each component can be stored individually under optimal conditions.

**Tab. 3: Examples for concentrated formulations and gels**

| Sample | | Z_{average} PI | | Z_{average} PI |
|---|---|---|---|---|
| DOTAP/CPT 90/10 15 mM 1 % Trehalose | After extrusion | 167 0.14 | Concentrated to 7% of original volume, and then brought back to the previous conentration | 166 0.24 |
| DOTAP/CPT 85/15 15 mM 1 % Trehalose | After extrusion | 180 0.12 | Concentrated to 7% of original volume, and then brought back to the previous conentration | 160 0.16 |
| 1. Adding of 3mM DOTAP in Ethanol to 1mM CPT-carboxylate in H₂O. (Drug/lipid ratio 1/9) | Final Concentration = 10 mM | 111 0.428 | Final Concentration = 20 mM | 67.6 0.342 |
| 2. Solvent evaporation | | | | |
| 1. Adding of 6 mM DOTAP in Ethanol to 2 mM CPT-carboxylate in H₂O. (Drug/lipid ratio 1/9) | Dilution from 100mM to 10mM | 93 0.619 | | |
| 2. Solvent evaporation. Final concentration 100 mM and 500 mM | | | | |

### 5. Mixing of pure lipid liposomes and drug solution directly before use

CPT containing liposomes can be prepared by mixing of an aqueous solution of CPT carboxylate with a suspension of cationic liposomes from pure lipids. Further, lyophilisates of cationic liposome suspensions from pure lipids can be reconstituted with an aqueous solution of CPT carboxylate to form ready to use liposomal formulations of CM-CT. This has the advantage that liposome suspension and CPT-carboxylate solution can be prepared and stored separately. The liposome suspension can be stored as it is or it can be lyophilised. The CPT-carboxylate can be stored as a solution, as a dry salt or as a lyophilisate. As an example a 30 mM suspension of liposomes from 100% DOTAP in 10% trehalose was lyophilised. Subsequently, the lyophilisate was reconstituted with 2.088 ml of a 1.5 mM solution of CPT carboxylate. Further, 250 µl of a 100 mM tris/HCl buffer solution, pH 7.5, was added. Physico-chemical measurements of the efficacy of insertion yielded a similar fraction of liposomal CPT as for formulations which had been prepared by the procedures as described before. The size distribution of the liposomes was not significantly affected.

### 6. Cationic polymer bound CPT

CPT binding to cationic polymers is achieved by mixing a solution of CPT-carboxylate and of the polymer. The solutions can be in water and/or in organic solvents, depending on the experimental necessities and the type of the polymer.
A typical type of polymers are polyelectrolytes, such as polyallyamine polyethyleneimine or polysaccarides (chitosan). The components are provided in the aqueous phase, containing, optionally buffers, ions and cryoprotectants. The polyelectrolytes are deposited onto oppositely charged surfaces and nanoparticles by physisorption. By repetitive deposition of positively and negatively charged polymer (such as polyallyamine or polyallyamine/CPT and polystyrene sulfonate) organized layered structures are obtained. For the deposition procedure, standard protocols are applied (Radtchenko et al. 2000) With this technique, nanoparticles, comprising CPT/polyelectrolyte, are assembled in a controlled way.

### 7. Physico-chemical characterization

### a. Fundamental aspects of CPT interaction with cationic lipids

For the present invention the interaction between CPT and the cationic organic molecules is fundamental. Therefore, the binding of CPT-carboxylate to lipids was characterized by a variety of methods. Particularly, the binding properties to DOTAP and to DOPC were compared, in order to highlight the advantages of the present invention with respect to other, earlier approaches.

Liposomal suspensions were prepared by ethanol injection of a lipid solutions into aqueous solutions of CPT-carboxylate, and the suspensions were dialized in order to remove free, non-liposomal CPT. The amount of the liposomal and the released CPT were determined by UV-vis spectroscopy. In Fig. 8, the results with DOTAP and DOPC, under identical conditions are shown. The concentration of the CPT which remained in the liposomal suspension is depicted as a function of dialysis time. As can be seen, virtually all of the CPT from the DOPC suspension is released in a short time scale, while for DOTAP liposomes, more that 60 % of the CPT remains with the liposomal suspension. This demonstrates, that only with DOTAP, but not with DOPC an attractive interaction and an effect of liposomal stabilization can be determined for the CPT-carboxylate.

In order to get further information on the CPT-lipid interaction form an independent method, fluorescence anisotropy measurements were performed. Colloidal suspensions from DOTAP and from DOPC together with CPT in different molecular forms were made up. The fluorescence anisotropy was taken as a measure for the binding strength of CPT to the liposome. In Fig. 9 the results of various measurements are shown. As expected, for the CPT-carboxylate solution without lipid, the anisotropy was zero, and as well with DOPC liposomes, the anisotropy was rather small. On the contrary, in all three experiments with DOTAP, the anisotropy was much higher, indicating CPT-carboxylate binding to the liposome. Depending on the mode of preparation, the anisotropy was different directly after formulation, but in all cases the same equilibrium value was reached after few hours. The highest anisotropy was found for the experiment where the liposomes were made by ethanol injection into aqueous solution of CPT, while when the liposomes were made with the CPT-lactone by the film method, it was significantly lower. This indicates, that in fact, the carboxylate form of CPT is necessary for efficient binding to the cationic lipid and for the formation of the CM-CPT containing liposomes.

This conjecture is clearly supported by the data depicted in Fig. 10. Fluorescence anisotropy measurements from liposomes with two different cationic lipids, DOTAP and DMTAP, and two different CPT drugs (CPT and 10-OH-CPT) are compared. With DMTAP the measurements were performed at room temperature and at 40°C, i.e. below and above the liquid crystalline/gel phase transition of the DMTAP. At low temperatures, the hydrophobic chains of the DMTAP are in a solid like state (gel phase), while at 40° they are in a fluid like (liquid crystalline) phase. DOTAP is already at room temperature in the fluid-like state. As can be seen, the anisotropies with DOTAP and DMTAP at 40°C are similar, while with DOTAP at RT it is significantly lower. This indicates, that it is favourable for efficient binding to the liposome, if the hydrocarbon chains are in a fluid like state, independently if the chains comprise double bonds or not. The comparison between CPT and 10-OH-CPT shows, that these conjectures are not only valid for CPT but as well for CPT derivates. In the present case, as due to the molecular properties of 10-OH-CPT an event higher binding efficacy is observed.

Summarizing, these data indicate consistently, that for highest binding efficiency of CPT to a lipid, it is necessary to provide the CPT in the carboxylate form, and to use a positively charged lipid as a counter molecule. The resulting CM-CPT can be clearly individualized and characterized by its physico-chemical properties. On the contrary, no such CM-CPT aggregate is formed with anionic or neutral lipids. This holds as well if the latter contain unsaturated fatty acids.

### b. Characterization of the liposomal formulations

All stages of formulation are monitored by HPLC analysis (lipid +drug) and by UV-vis and fluorescence spectroscopy. In HPLC analysis a slight loss of material after extrusion can be observed. UV-vis and fluorescence spectroscopy serves as a qualitative means in order to control formulation efficacy.

It is well known, that the spectrum of CPT depends strongly on the molecular state and on the local environment. Therefore, qualitatively, the spectra of the lactone form, the carboxylate and the liposomal CPT can be distinguished from the shape of the spectra (Fig. 4, Fig. 5). In Fig. 4 the excitation and emission spectra of the lipid-bound CPT and the free drug in organic solvent (Chloroform/Methanol) are shown. In the fluorescence spectrum, binding to the lipid induces a significant red-shift and a change of the peak shape. The changes of the absorption spectrum are most clearly seen in Fig. 5. The upper curve gives the spectrum of lipid-bound CPT, and subsequently spectra of free CPT under different conditions are shown. The second and the third curve correspond to the lactone form of the molecule, and the fourth and the fifth curve correspond to the lactone form of the molecule.
Quasi-elastic light scattering (Zetasizer 1000 and Zetasizer 3000, Malvern Instruments, Herrenberg, Germany) was measured in order to determine the size distribution of the liposomes. The zeta potential was determined by a Zetasizer 3000 (Malvern Instruments). In Tab.1, the results from the characterization of some exemplary formulations are shown.

### 8. In vitro determination of the cytostatic efficacy of LipoCam

The efficacy of a cytostatic drug is determined in vitro by analysing the decrease of cell viability in correlation to the drug concentration. The drug concentration at which cell viability is inhibited to 50 % ("IC₅₀") is used as index for the inhibitory potential of a respective drug. The higher the cytostatic potential, the lower is the IC50 value. Drugs with high inhibitory potential have IC₅₀ values in the nM range. Here we compared 3 formulations of liposomally encapsulated camptothecin carboxylate (RM544=Lipocam I, RM541 = Lipocam II, RM542 = Lipocam III) with free carboxylate (RM543).

### Principle:

A suitable cell line (i.e. endothelial or tumor cell line) is seeded at a constant densities in four 24-well plates. After one or two days, a series of 11 consecutive drug dilutions is added to cover the range between 0 - 5000 nM final drug concentration. Each individual concentration is measured in 2 wells independently to increase the accuracy of the assay. Two wells without drug serve as control. The cells are incubated at optimal growth conditions (5-5.5 % CO₂, 37°C, ∼ 90% humidity). After 72 h, the cell viability in each well is determined by measuring the activity of mitochondrial dehydrogenases (MTT assay). In viable cells the MTT substrate is converted to a blue, cell impermeable dye (Formazan). After around 1 h, the medium is removed, cells are lysed with isopropanol/0,04% HCl and the amount of the blue Formazan quantitated in an ELISA reader at 550 nm (measured against lysis buffer as blank). The experiment is evaluated using the Sigma Plot analysis software by plotting the mean OD₅₅₀ₙₘ value (of the 2 wells containing the identical drug concentration) against the respective drug concentration. The IC₅₀ concentration is taken at the half-maximal OD₅₅₀ₙₘ value. A high inhibitory potential results in a low IC₅₀ value.

### Experiment:

2x 10⁴/cm² Ea.Hy926 cells (transformed human endothelial line) are seeded into four 24-well plates and cultivated over night. At day 1, a series of eleven 5x concentrated master dilutions of each camptothecin formulation in cell culture medium is prepared (25000, 10000, 5000, 2500, 1250, 500, 250, 50, 25, 12.5, 5 nM). Having prepared all dilutions, the culture medium is removed from the cells and 400 µl of fresh culture medium + 100 µl of the respective master drug concentration is added (each concentration to 2 wells). This results in a 1:5 dilution of the master series (final drug concentrations between 1 nm and 5000 nM). To two wells no drug is added (controls). The plates are cultivated further 72 h. After this incubation period, the medium is replaced by fresh medium containing the MTT substrat and incubated for 1 h. The medium is removed, the cells are lysed in isopropanol/0.04% HCl and the OD at 550 nm is measured.

### Result:

As demonstrated in Fig. 11, all three LipoCam formulations (RM541, RM542, RM544) display a comparable or better growth inhibition curve than free camptothecin carboxylate. The IC₅₀ values are around 25-30 nM. Therefore, liposomally encapsulated camptothecin carboxylate has a high cell inhibitory efficacy in vitro.

### 9. Therapeutic efficacy of LinoCam in A-375 melanoma of nude mice

NMRI-nude mice were purchased from Elevage Janvier and housed in isolated ventilated cages under save environmental conditions (SPF facility, 22°C, 30 - 70% humidity, 12 h light/dark cycle) with food and water ad libitum. Experimental design was reviewed and approved by local government.

Tumor cells (A-375 human melanoma cell line, ATCC Nr.: CRL-1619) were grown as described in the data sheet supplied by ATCC. Tumor cells (5 x 10⁶ in PBS) were inoculated s.c. in the right dorsal flank of mice in a volume of 50 µl on day O.

Mice were assigned to the experimental groups (8 animals per cage), housed and handled (including monitoring of the body weight gain) at least five days before tumor inoculation (= day -6 to 0). Treatment begins after tumors reached a volume of approximately 100 mm³. Drugs were given by iv injection, three times a week (Mo, Wed, Fri) for the following three weeks at equitoxic doses. Drugs were prepared as described earlier. Equitoxic doses of the compounds were determined in separate experiments, where toxicity was evaluated based on haematological parameters, body weight and animal clinical observations (see Tab. 3). The solutions were administered slowly in a volume of -10 µl/g body weight.

Animals were clinically monitored during the whole experiment, monitoring of tumor size was performed three times a week. The tumor dimensions were measured by caliper and the tumor size was calculated according to the following formula: V = π L W² / 6 (L = greatest length, W = width of perpendicular axis). The body weight of individual animals was monitored at least twice during handling period, after tumor inoculation and after start of treatment. EDTA blood was collected from the retrobulbar plexus at three different points: during handling, tumor staging and in the middle of treatment from 4 animals of all treatment groups for hematology. The number of red and white blood cells and platelets were determined using an automated cell counter (Abbott Cell Dyn 3500).

Whereas tumors in the control group showed a rapid and progressive tumor growth, all drug formulations - applied at equitoxic doses - showed an anti-tumor effect (Fig. 12). The most effective formulation was LipoCam III, with total tumor regression in most animals. LipoCam I and LipoCam II showed also a partial tumor regression with a slow re-growth of tumors beginning in the second half of the treatment period. CPT-Na-Carboxylate showed only a retardation as compared to the control group.

### 10. Human Therapy Treatment Protocols

This example is concerned with human treatment protocols using the formulations disclosed. Treatment will be of use for diagnosing, preventing and/or treating various human diseases and disorders associated with enhanced angiogenic activity. It is considered to be particularly useful in anti-tumor therapy, for example, in treating patients with solid tumors and hematological malignancies or in therapy against a variety of chronic inflammatory diseases such as psoriasis.

A feature of the invention is that several classes of diseases and/or abnormalities are treated without directly treating the tissue involved in the abnormality e.g., by inhibiting angiogenesis the blood supply to a tumor is cut off and the tumor is killed without directly treating the tumor cells in any manner.

Methods of treating such patients using lipid:drug complexes have already been formulated, for example, see document incorporated herein by reference. It is contemplated that such methods may be straightforwardly adapted for use with the method described herein. As discussed above, other therapeutic agents could be administered either simultaneously or at distinct times. One may therefore employ either a pre-mixed pharmacological composition or "cocktail" of the therapeutic agents, or alternatively, employ distinct aliquots of the agents from separate containers.

The various elements of conducting a clinical trial, including patient treatment and monitoring, will be known to those of skill in the art in light of the present disclosure.

For regulatory approval purposes, it is contemplated that patients chosen for a study would have failed to respond to at least one course of conventional therapy and would have objectively measurable disease as determined by physical examination, laboratory techniques, or radiographic procedures. Such patients would also have no history of cardiac or renal disease and any chemotherapy should be stopped at least 2 weeks before entry into the study.

Prior to application, the formulations can be reconstituted in an aqueous solution in case the formulation was freeze dried. Again, the required application volume is calculated from the patient's body weight and the dose schedule.

The disclosed formulations may be administered over a short infusion time. The infusion given at any dose level should be dependent upon the toxicity achieved after each. Hence, if Grade II toxicity was reached after any single infusion, or at a particular period of time for a steady rate infusion, further doses should be withheld or the steady rate infusion stopped unless toxicity improved. Increasing doses should be administered to groups of patients until approximately 60% of patients showed unacceptable Grade III or IV toxicity in any category. Doses that are 2/3 of this value would be defined as the safe dose.

Physical examination, tumor measurements, and laboratory tests should, of course, be performed before treatment and at intervals of about 3-4 weeks later. Laboratory tests should include complete blood counts, serum creatinine, creatine kinase, electrolytes, urea, nitrogen, SGOT, bilirubin, albumin, and total serum protein.

Clinical responses may be defined by acceptable measure or changes in laboratory values e.g. tumormarkers. For example, a complete response may be defined by the disappearance of all measurable disease for at least a month. Whereas a partial response may be defined by a 50% or greater reduction.

All of the compositions and methods disclosed and claimed herein can be made and executed without undue experimentation in light of the present disclosure. While the compositions and methods of this invention have been described in terms of preferred embodiments, it will be apparent to those of skill in the art that variations may be applied to the composition, methods and in the steps or in the sequence of steps of the method described herein without departing from the concept, spirit and scope of the invention. More specifically, it will be apparent that certain agents which are both chemically and physiologically related may be substituted for the agents described herein while the same or similar results would be achieved. All such similar substitutes and modifications apparent to those skilled in the art are deemed to be within the spirit, scope and concept of the invention as defined by the appended claims.

Some variation in dosage will necessarily occur depending on the condition of the subject being treated. The person responsible for administration will, in any event, determine the appropriate dose for the individual subject. Moreover, for human administration, preparations should meet sterility, pyrogenicity, general safety and purity standards as required by FDA Office of Biologics standards.

### Administration and dosing

The present invention includes a method of delivery of a pharmaceutically effective amount of the inventive formulation of a camptothecin drug to an angiogenic vascular target site of a subject in need thereof. A "subject in need thereof" thereby refers to a mammal, e. g. a human.

The route of administration comprises peritoneal, parenteral or topic administration and the formulations are easily administered in a variety of dosage forms such as injectable solutions, drug release capsules and the like.

For use with the present invention the term "pharmacologically effective amount" of a compound administered to a subject in need thereof (which may be any animal with a circulatory system with endothelial cells which undergo angiogenesis) will vary depending on a wide range of factors. For example, it would be necessary to provide substantially larger doses to humans than to smaller animal. The amount of the compound will depend upon the size, age, sex, weight, and condition of the patient as well as the potency of the substance being administered. Having indicated that there is considerable variability in terms of dosing, it is believed that those skilled in the art can, using the present disclosure, readily determine appropriate dosing by first administering extremely small amounts and incrementally increasing the dose until the desired results are obtained. Although the amount of the dose will vary greatly based on factors as described above, in general, the present invention makes it possible to administer substantially smaller amounts of any substance as compared with delivery systems which target the surrounding tissue e. g., target the tumor cells themselves.

The pharmaceutically effective amount of a therapeutic agent as disclosed herein depends on the kind and the type of action of the agent. For the examples mentioned herein, it is within the range of about about 0.1 to 20 mg/kg in humans. Typically, for camptothecin drugs, doses in the order of about 5 mg/kg are applied.

### 9. Preparation of Lipocam formulations

Procedures for the production of LipoCam formulations
a) 30 mM DOTAP/CPT molar ratio 95/5
b) 30 mM DOTAP/CPT molar ratio 97.5/2.5
c) 30 mM DOTAP/PEG-DOPE/CPT molar ratio 90/5/5
   are described. All formulations are realized by the same protocol consisting of a sequence of defined formulations steps. In order to obtain the different formulations, the amounts of the components are varied. Typical volumina of the final suspensions were 10-100 ml.

### CPT-carboxylate solution

The carboxylate salt of a CPT drug is obtained by exposing the CPT drug to an alkaline environment with a pH above 9. 2 mg/ml of CPT drug are dissolved in 25% NH₄OH. Subsequently the solvent is evaporated. In this way, the pure ammonium salt of the CPT drug-carboxylate is obtained. The salt is dissolved in water (ca. 2-3 mM) and a suitable amount is added to a trehalose Tris/HCl, pH 7.5, stock solution to obtain a final concentration of 10% trehalose (w/v), 10 mM Tris and x mM CPT where x is 1.5 for example a) and c), and 0.75 for example b).

### Ethanol injection

A 400 mM ethanolic solution of the lipid or lipid mixture is injected into the CPT-carboxylate solution under vigorous stirring. The solution contains only DOTAP for example a) and b) and it contains DOTAP/PEG-DOPE in the molar ratio 95/5 for example c). Thus, by a self-assembly process, polydisperse multilamellar vesicles are formed.

### Extrusion

In order to obtain unilamellar vesicles of uniform size, the liposome suspensions were extruded five times through membranes of 200 nm pores size (Osmonics Inc., Poretics, polycarbonate 0.2 Microns). An additional optional step in case of animal studies was sterile filtration (Millipak 200, 0.22 µm).

The formulations were checked by HPLC for composition. Size distributions were controlled by quasi-elastic light scattering (Malvern, Herrenberg, Germany).

| Formulation | Composition DOTAP/PEG/CPT | Size distribution Zₐᵥₑ (PI) | DOTAP theor | DOTAP found | CPT theor | CPT found/ |
|---|---|---|---|---|---|---|
| LipoCam IV | 95/0/5 | 214 nm (0.187) | 28.5 | 27.5 | 0.523 | 0.474 |
| LipoCam VI | 97.5/0/2.5 | 224 nm (0.137) | 29.25 | 27.2 | 0.261 | 0.213 |
| PEG- LipoCam IV | 95/5/5 | 242 nm (0.129) | 28.5 | 27.4 | 0.523 | 0.45 |

### Results form HPLC and PCS analysis

### 10. Therapeutic efficacy of LipoCam IV and VI in A-375 melanoma of nude mice

NMRI-nude mice were purchased from Elevage Janvier and housed in isolated ventilated cages under save environmental conditions (SPF facility, 22°C, 30 - 70% humidity, 12 h light/dark cycle) with food and water ad libitum. Experimental design was reviewed and approved by local government.

Tumor cells (A-375 human melanoma cell line, ATCC Nr.: CRL-1619) were grown as described in the data sheet supplied by ATCC. Tumor cells (5 x 10⁶ in PBS) were inoculated s.c. in the right dorsal flank of mice in a volume of 50 µl on day 0.

Mice were assigned to the experimental groups (8 animals per cage), housed and handled (including monitoring of the body weight gain) at least five days before tumor inoculation (= day -6 to 0). Treatment begins after the tumors reached a volume of approximately 80 mm3. Drugs were given by iv injection, three times a week (Mo, Wed, Fri) for the following three weeks. Drugs were prepared as described earlier (see Example 9). Toxicity was evaluated based on hematological parameters, body weight and animal clinical observations. The solutions were administered slowly in a volume of ∼ 5 µl/g body weight.

Animals were clinically monitored during the whole experiment, monitoring of tumor size was performed three times a week. The tumor dimensions were measured by caliper and the tumor size was calculated according to the following formula: V = πL W² / 6 (L = greatest length, W = width of perpendicular axis). The body weight of individual animals was monitored at least twice during handling period, after tumor inoculation and after start of treatment. EDTA blood was collected from the retrobulbar plexus at three different points: during handling, tumor staging and in the middle of treatment from 4 animals of all treatment groups for hematology. The number of red and white blood cells and platelets were determined using an automated cell counter (Abbott Cell Dyn 3500).

As shown in Fig. 13, treatment with LipoCam IV (2 mg/kg) and LipoCam VI (1 mg/kg) was far more effective as compared to the free drug CPT-carboxylate at the respective doses. Notably, treatment with LipoCam at both doses resulted in beginning tumor regression even after the first application. The therapeutic efficacy of the LipoCam formulations and the free drug was dose-dependent. The antitumor efficacy of the formulations is in the order LipoCam IV (2 mg/kg) > LipoCam VI (1 mg/kg) > CPT-carboxylate (2 mg/kg) > CPT-carboxylate (1 mg/kg). In comparison, tumors in the control group showed a rapid and progressive tumor growth, reaching a mean tumor volume of > 1000 mm³ on day 30 after tumor cell inoculation.

### 11. Therapeutic efficacy of LicoCam IV and PEG-LipoCam IV in A-375 melanoma of nude mice

NMRI-nude mice were purchased from Elevage Janvier and housed in isolated ventilated cages under save environmental conditions (SPF facility, 22°C, 30 - 70% humidity, 12 h light/dark cycle) with food and water ad libitum. Experimental design was reviewed and approved by local government.

Tumor cells (A-375 human melanoma cell line, ATCC Nr.: CRL-1619) were grown as described in the data sheet supplied by ATCC. Tumor cells (5 x 10⁶ in PBS) were inoculated s.c. in the right dorsal flank of mice in a volume of 50 µl on day 0.

Mice were assigned to the experimental groups (8 animals per cage), housed and handled (including monitoring of the body weight gain) at least five days before tumor inoculation ( = day -6 to 0). Treatment begins after the tumors reached a volume of approximately 300 mm³. Drugs were given by iv injection, three times a week (Mo, Wed, Fri) for the following three weeks. Drugs were prepared as described earlier (see Example 9). Toxicity was evaluated based on hematological parameters, body weight and animal clinical observations. The solutions were administered slowly in a volume of ∼ 5 µl/g body weight.

Animals were clinically monitored during the whole experiment, monitoring of tumor size was performed three times a week. The tumor dimensions were measured by caliper and the tumor size was calculated according to the following formula: V = πL W² / 6 (L = greatest length, W = width of perpendicular axis). The body weight of individual animals was monitored at least twice during handling period, after tumor inoculation and after start of treatment. EDTA blood was collected from the retrobulbar plexus at three different points: during handling, tumor staging and in the middle of treatment from 4 animals of all treatment groups for hematology. The number of red and white blood cells and platelets were determined using an automated cell counter (Abbott Cell Dyn 3500).

In Fig. 14, the therapeutic efficacy of LipoCam IV (2 mg/kg) and PEG-LipoCam IV (2 mg/kg) was compared. Both formulations strongly reduced the tumor growth rate, with a more pronounced antitumor effect for the non-PEGylated formulation. In comparison, tumors in the control group showed a rapid and progressive tumor growth, reaching a mean tumor volume of - 3000 mm³ on day 35 after tumor cell inoculation.

### REFERENCES

Hertzberg, et. al. (1989) "Modification of the hydroxy lactone ring of camptothecin: inhibition of mammalian topoisomerasel and biological activity" Journal of Medical Chemistry, 32, 715.
Hsiang, Y. H. and L. F. Liu (1988). "Identification of mammalian DNA topoisomerase I as an intracellular target of the anticancer drug camptothecin." Cancer Res 48(7): 1722-6.
Kehrer, D. F., O. Soepenberg, et al. (2001). "Modulation of camptothecin analogs in the treatment of cancer: a review." Anticancer Drugs 12(2): 89-105.
Lewis, R. N., S. Tristram-Nagle, et al. (2001). "The thermotropic phase behavior of cationic lipids: calorimetric, infrared spectroscopic and X-ray diffraction studies of lipid bilayer membranes composed of 1,2-di-O-myristoyl-3-N,N,N-trimethylaminopropane (DM-TAP)." Biochim Biophys Acta 1510(1-2): 70-82.
Lundberg, B. B. (1998). "Biologically active camptothecin derivatives for incorporation into liposome bilayers and lipid emulsions." Anticancer Drug Des 13(5): 453-61.
Moertel, C. G., A. J. Schutt, et al. (1972). "Phase II study of camptothecin (NSC-100880) in the treatment of advanced gastrointestinal cancer." Cancer Chemother Rep 56(1): 95-101.
Muggia, F. M., P. J. Creaven, et al. (1972). "Phase I clinical trial of weekly and daily treatment with camptothecin (NSC-100880): correlation with preclinical studies." Cancer Chemother Rep 56(4): 515-21.
Radtchenko IL, Sukhorukov GB, Leporatti S, Khomutov GB, Donath E, Mohwald (2000) "Assembly of Alternated Multivalent Ion/Polyelectrolyte Layers on Colloidal Particles. Stability of the Multilayers and Encapsulation of Macromolecules into Polyelectrolyte Capsules" J Colloid Interface Sci. 230(2):272-280.
Wall, M. E. and M. C. Wani (1996). "Camptothecin and taxol: from discovery to clinic." J Ethnopharmacol 51(1-3): 239-53; discussion 253-4.
Wall, M. E., M. C. Wani, et al. (1966). "Plant antitumor agents. I. The isolation and structure of camptothecin, a novel alkaloidal leukemia and tumor inhibitor from camptotheca acuminata 1,2." Journal of the American Chemical Society 88(16): 3888-3890.
Zunino F, et al. (2002) Current status and perspectives in the development of camptothecins. Curr Pharm Des. 8(27):2505-20.

**Table 4 (Appendix)**

| **Nr.** | **Experiment** | **Composition** | **mM** | **Buffer** |
|---|---|---|---|---|
| 1 | RM505 | Dotap/Dopc/CPT 50/49.910.1 | 25 | Tris/5% Glucose, pH 9 |
| 2 | RM506 | Dotap/Dopc/CPT 50/49.8/0.2 | 25 | Tris/5% Glucose, pH 9 |
| 3 | RM507 | Dotap/Dopc/CPT 50/49.7/0.3 | 25 | Tris/5% Glucose, pH 9 |
| 4 | RM508 | Dotap/Dopc/CPT 50/49.5/0.5 | 25 | Tris/5% Glucose, pH 9 |
| 5 | RM509 | Dotap/DopdCPT 50/49/1 | 25 | Tris/5% Glucose, pH 9 |
| 6 | RM476 RM484 | Dotap/DopdCPT 50/47/3 | 25 | 5% Glucose |
| 7 | RM485 RM490 | Dotap/Dopc/CPT 50/47/3 | 25 | Tris/5% Glucose, pH 9 |
| 8 | SN5 | Dotap/DopGCPT 50/45/5 | 25 | Tris/5% Glucose, pH 9 |
| 9 | SN8 | Dotap/DopdCPT 50142.5/7.5 | 25 | Tris/5% Glucose, pH 9 |
| 10 | SN7 | Dotap/Dopc/CPT 50/40/10 | 25 | Tris/5% Glucose, pH 9 |
| 11 | SN13 | Dotap/Dopc/CPT 50/37.5/12.5 | 25 | Tris/5% Glucose, pH 9 |
| 12 | SN6 | Dotap/Dopc/CPT 50/35/15 | 25 | Tris/5% Glucose, pH 9 |
| 13 | RM532 | Dotap/Dopc/CPT 55/40/5 | 25 | Tris/5% Glucose, pH 8.7 |
| 14 | RM525 RM544 | Dotap/Dopc/CPT 55/40/5 | 25 | Tris/5% Glucose, pH 8.8 |
| 15 | RM518 | Dotap/Dopc/CPT 55/40/5 | 25 | Tris/5% Glucose, pH 9 |
| 16 | IK15 | Dotap/Dopc/CPT 60/30/10 | 25 | Tris/5% Glucose, pH 7 |
| 17 | IK14 | Dotap/Dopc/CPT 60/30/10 | 25 | Tris/5% Glucose, pH 8 |
| 18 | RM533 | Dotap/DopcICPT 60/30/10 | 25 | Tris/5% Glucose, pH 8.7 |
| 19 | RM526 RM541 | Dotap/Dopc/CPT 60/30/10 | 25 | Tris/5% Glucose, pH 8.8 |
| 20 | RM495 RM519 | Dotap/Dopc/CPT 60/30/10 | 25 | Tris/5% Glucose, pH 9 |
| 21 | RM496 | Dotap/DopdCPT 62.5/25/12.5 | 25 | Tris/5% Glucose, pH 9 |
| 22 | RM497 | Dotap/Dopc/CPT 65/20/15 | 25 | Tris/5% Glucose, pH 9 |
| 23 | UF4 UF11 | Rh-Oope/Dotap/Dopc/CPT 1/55/39/5 | 15 | 10 mM Tris/5% Glucose |
| 24 | UF9 | Rh-Dope/Dotap/Dopc/CPT 5/55/35/5 | 15 | 10 mM Tris/5% Glucose |
| 25 | UF5 UF11 | Rh-Dope/Dotap/CPT 1/89/10 | 15 | 10 mM Tris/5% Glucose |
| 26 | UF8 | Rh-Dope/Dotap/CPT 5/85/10 | 15 | 10 mM Tris/5% Glucose |
| 27 | SN14 | Dotap/Chol/CPT 55/40/5 | 25 | Tris/5% Glucose, pH 9 |
| 28 | SN15 | Dotap/Chol/CPT 60/30/10 | 25 | Tris/5% Glucose, pH 9 |
| 29 | UF57.UF61 UF74 | Dotap/Dope-Peg/CPT 90/5/5 | 25 | 10% Trehalose, pH 7.5 |
| 30 | UF42 | Dotap/Dope-Peg/CPT 85/5/10 | 25 | 10% Trehalose, pH 7.5 |
| | | | | |

| **Nr.** | **Experiment** | **Composition** | **mM** | **Buffer** |
|---|---|---|---|---|
| 31 | UF71 | Dotap/CPT 97.5/2.5 | 30 | 10% Trehalose, pH 7.5 |
| 32 | UF20 | Dotap/CPT 95/5 | 10 | 10% Trehalose |
| 33 | UF23 | Dotap/CPT 95/5 | 10 | 10% Trehalose, pH 6.5 |
| 34 | UF21 | Dotap/CPT 95/5 | 10 | 10% Trehalose, pH 7 |
| 35 | UF51 | Dotap/CPT 95/5 | 10 | 10% Trehalose, pH 7.5 |
| 36 | RM626 | Dotap/CPT 95/5 | 15 | 10% Trehalose |
| 37 | UF67 | Dotap/CPT 95/5 | 25 | 10% Trehalose |
| 38 | UF53 UF60 | Dotap/CPT 95/5 | 25 | 10% Trehalose, pH 7.5 |
| 39 | UF59 | Dotap/CPT 95/5 | 50 | 10% Trehalose, pH 7.5 |
| 40 | UF62, UF63 RM708 | Dotap/CPT 95/5 | ca.500 | 10% Trehalose, pH 7.5 |
| 41 | UF14 | Dotap/CPT 90/10 | 10 | 5% Glucose, pH 8 |
| 42 | UF48 | Dotap/CPT 90/10 | 10 | 1% Trehalose, pH 7.5 |
| 43 | UF20 | Dotap/CPT 90/10 | 10 | 10% Trehalose |
| 44 | UF23 | Dotap/CPT 90/10 | 10 | 10% Trehalose, pH 6.5 |
| 45 | UF21 | Dotap/CPT 90/10 | 10 | 10% Trehalose, pH 7 |
| 46 | UF41 UF44 | Dotap/CPT 90/10 | 10 | 10% Trehalose, pH 7.5 |
| 47 | RM567 | Dotap/CPT 90/10 | 15 | Tris/10% Trehalose, pH 8.7 |
| 48 | RM554, RM573 RM584, RM591 | Dotap/CPT 90/10 | 15 | Tris/5% Glucose, pH 8.8 |
| 49 | UF28 | Dotap/CPT 90/10 | 15 | Reinstwasser |
| 50 | UF29 | Dotap/CPT 90/10 | 15 | 1% Trehalose |
| 51 | RM623, RM620 RM612, RM622 | Dotap/CPT 90/10 | 15 | 10% Trehalose |
| 52 | RM613 | Dotap/CPT 90/10 | 15 | 10% Trehalose, pH 6.5 |
| 53 | RM619, UF33 | Dotap/CPT 90/10 | 15 | 10% Trehalose, pH 7.5 |
| 54 | RM624 | Dotap/CPT 90/10 | 20 | 10% Trehalose |
| 55 | IK17 | Dotap/CPT 90/10 | 25 | Tris/5% Glucose, pH 7 |
| 56 | IK16 | Dotap/CPT 90/10 | 25 | Tris/5% Glucose, pH 8 |
| 57 | RM534 | Dotap/CPT 90/10 | 25 | Tris/5% Glucose, pH 8.7 |
| 58 | RM527 RM542 | Dotap/CPT 90/10 | 25 | Tris/5% Glucose, pH 8.8 |
| 59 | RM501 RM520 | Dotap/CPT 90/10 | 25 | Tris/5% Glucose, pH 9 |
| 60 | RM625 | Dotap/CPT 90/10 | 25 | 10% Trehalose |
| 61 | UP51.UFS5 UF58 | Dotap/CPT 90/10 | 25 | 10% Trehalose, pH 7.5 |
| 62 | UF49 | Dotap/CPT 90/10 | ca.100 | 10% Trehalose, pH 7.5 |

| **Nr.** | **Experiment** | **Composition** | **mM** | **Buffer** |
|---|---|---|---|---|
| 63 | UF56 | Dotap/CPT 90/10 | ca. 500 | |
| 64 | RM627 UF20. UF22, UF24 | Dotap/CPT 85/15, | 10 | 10% Trehalose |
| 65 | UF23 | Dotap/CPT 85/15 | 10 | 10% Trehalose, pH 6.5 |
| 66 | UF21 | Dotap/CPT 85/15 | 10 | 10% Trehalose, pH 7 |
| 67 | UF54 | Dotap/CPT 85/15 | 10 | 10% Trehalose, pH 7.5 |
| 68 | UF28 | Dotap/CPT 85/15 | 15 | Reinstwasser |
| 69 | UF29 | Dotap/CPT 85/15 | 15 | 1% Trehalose |
| 70 | RM627 UF22 | Dotap/CPT 85/15 | 15 | 10% Trehalose |
| 71 | UF22 | Dotap/CPT 85/15 | 20 | 10% Trehalose |
| 72 | UF22 | Dotap/CPT 85/15 | 25 | 10% Trehalose |
| 73 | UF20 | Dotap/CPT 80/20 | 10 | 10% Trehalose |
| 74 | UF23 | Dotap/CPT 80/20 | 10 | 10% Trehalose, pH 6.5 |
| 75 | UF21 | Dotap/CPT 80/20 | 10 | 10% Trehalose, pH 7 |
| 76 | UF19 | Dotap/CPT 80/20 | 10 | 10% Trehalose, pH 7.5 |
| 77 | RM628 | Dotap/CPT 80/20 | 15 | 10% Trehalose |
| 78 | RM502 | Dotap/CPT 80/20 | 25 | Tris/5% Glucose, pH 9 |
| 79 | UF16 | Dmtap/CPT 90/10 | 10 | 5% Trehalose, pH 8 |
| 80 | UF16 | Dstap/CPT 90/10 | 10 | 5% Trehalose, pH 8 |
| 81 | RM514 RM515 | Dopc/CPT 90/10 | 2.5 | Tris/5% Glucose pH 7.5 |
| 82 | RM516 | Dopc/CPT 90/10 | 2.5 | Tris/5% Glucose pH.9 |
| 83 | UF35 | Dopc/CPT 90/10 | 15 | 10% Trehalose, pH 7.6 |
| 84 | UF75 | Dotap/10-Hydroxy-CPT | 25 | 10% Trehalose, pH 7.5 |
| 85 | UF184 | Dotap/SN38 | 25 | 10% Trehalose, pH 7.5 |
| 86 | UF76 | Dotap/Irinotecan 95/5 | 25 | 10% Trehalose, pH 7.5 |
| 87 | UF77 | Dotap/Topotecan 95/5 | 10 | 10% Trehalose, pH 7.5 |
| 88 | UF78 | otap/CPT-Oleat 95/5 | 25 | 10% Trehalose, pH 7.5 |

## Claims

1. A composition comprising the carboxylate form of a camptothecin drug associated with at least one cationic amphiphile and/or cationic polymer (cationic molecule) having a positive net charge wherein the molar ratio of the cationic molecule to the camptothecin drug is at least about 1:1 wherein said composition is substantially free of the lactone form of said camptothecin.

2. The composition of claim 1, wherein said cationic amphiphile is selected from lipids, lysolipids or pegylated lipids, preferably having a tertiary amino or quaternary ammonium group such as
N-[1-(2,3-diacyloxy)propyl]-N,N-dimethylamine or
N-[1-(2,3-diacyloxy)propyl]-N,N,N-trimethyl ammonium.

3. The composition of any one of claims 1 or 2, wherein said cationic polymer is a polyelectrolyte, acid such as polyallylamine or polyethylene imine, a polymeric sugar or a polyamine with a molecular weight between about 5 and about 500 kDa.

4. The composition of any one of the claims 1 to 3, further comprising at least one anionic and/or neutral amphiphile.

5. The composition of any one of claims 1 to 4, wherein said anionic and/or neutral amphiphile is selected from sterols or lipids such as cholesterol, phospholipids, lysolipids, lysophospholipids, sphingolipids or pegylated lipids with a negative or neutral net charge.

6. The composition of any one of the claims 4 to 5, wherein the neutral amphiphile is diacylphosphatidylcholine.

7. A colloidal nanoaggregate comprising a composition of any one of the claims 1 to 6.

8. The nanoaggregate of claim 7 having an overall positive charge.

9. The nanoaggregate of claim 7 or 8, further comprising at least one amphiphile which has a negative and/or neutral net charge (anionic and/or neutral amphiphile).

10. The nanoaggregate of any one of the claims 7 to 9, wherein said anionic and/or neutral amphiphile is selected from sterols or lipids such as cholesterol, phospholipids, lysolipids, lysophospholipids, sphingolipids or pegylated lipids with a negative or neutral net charge.

11. The nanoaggregate of any one of the claims 7 to 10, wherein the neutral amphiphile is diacylphosphatidylcholine.

12. The nanoaggregate of any one of the claims 7 to 11, comprising an excess of positively charged moieties of at least about 20 %, preferably at least about 30 % and most preferably at least about 40 % in the outer molecular layer.

13. The nanoaggregate of any one of the claims 7 to 12, which is present as an emulsion droplet, a micelle, a liposome, a nanoparticle or a nanocapsule.

14. The nanoaggregate of any one of the claims 7 to 13, comprising about 0.1 to about 50 mol% of a camptothecin drug or a derivative thereof.

15. The nanoaggregate of any one of the claims 7 to 14, further comprising a cryoprotectant which is selected from a sugar or an alcohol or a combination thereof such as trehalose, maltose, sucrose, glucose, lactose, dextran, mannitol or sorbitol.

16. A pharmaceutical preparation comprising a pharmaceutically effective amount of a composition comprising the carboxylate form of a camptothecin drug associated with at least one organic cationic molecule having a positive net charge wherein the molar ratio of the organic cationic molecule to the camptothecin drug is at least about 1:1, wherein said composition is substantially free of the lactone form of said camptothecin, or a colloidal nanoaggregate thereof, together with a pharmaceutically acceptable carrier, diluent and/or adjuvant.

17. A method of producing the colloidal nanoaggregate of any one of the claims 7 to 15, comprising the steps of
(a) providing a camptothecin drug, preferably as a salt,
(b) associating said camptotecin drug in its carboxylate form with a cationic amphiphile having a positive net charge and optionally at least one further amphiphile which has a positive, negative and/or neutral net charge, and
(c) forming a colloidal nanoaggregate.

18. The method of claim 17, wherein step b) and c) comprise forming said nanoaggregate by a homogenisation, a lipid film or by a solvent injection procedure.

19. The use of a pharmaceutical preparation of claim 16 for producing a medicament for treating and/or preventing cancer, inflammatory diseases, diabetic retinopathy, rheumatoid arthritis, dermatitis, psoriasis, stomach ulcers and macular degeneration.

## Patentansprüche

1. Zusammensetzung mit einer positiven Nettoladung, die die Carboxlatform eines Camptothecin-Wirkstoffs umfasst, welche mit mindestens einem kationischen Amphiphil und/oder kationischen Polymer (kationisches Molekül) assoziiert ist, in der das molare Verhältnis des kationischen Moleküls zum Camptothecin-Wirkstoff mindestens 1:1 beträgt, wobei die Zusammensetzung im Wesentlichen frei von der Lactonform des Camptothecins ist.

2. Die Zusammensetzung nach Anspruch 1, in der das kationische Amphiphil aus Lipiden, Lysolipiden oder pegylierten Lipiden ausgewählt ist, bevorzugt solchen die eine tertiäre Aminogruppe oder quaternäre Ammoniumgruppe aufweisen wie N-[1-(2,3-diacyloxy)propyl]-N,N-dimethlyamin oder N-[1-(2,3-diacyloxy)propyl]-N,N,N-trimethylammonium.

3. Die Zusammensetzung nach einem der Ansprüche 1 oder 2, in der das kationische Polymer ein Polyelektrolyt, Säure wie Polyallylamin oder Polyethylenimin, ein polymerer Zucker oder ein Polyamin mit einem Molekulargewicht zwischen etwa 5 und etwa 500 kD ist.

4. Die Zusammensetzung nach einem der Ansprüche 1 bis 3, die weiterhin mindestens ein anionisches und/oder neutrales Amphiphil umfasst.

5. Die Zusammensetzung nach Anspruch 4, in der das anionische und/oder neutrale Amphiphil aus Sterolen oder Lipiden ausgewählt ist, wie zum Beispiel Cholesterin, Phospholipiden, Lysolipiden, Lysophospholipiden, Sphingolipden oder pegylierten Lipiden mit einer negativen oder neutralen Nettoladung.

6. Die Zusammensetzung nach einem der Ansprüche 4 oder 5, in der das neutrale Amphiphil Diacylphosphatidylcholin ist.

7. Kolloidales Nanoaggregat, das eine Zusammensetzung nach einem der Ansprüche 1 bis 6 umfasst.

8. Das Nanoaggregat nach Anspruch 7 mit einer positiven Gesamtladung.

9. Das Nanoaggregat nach Anspruch 7 oder 8, das weiterhin mindestens ein Amphiphil umfasst, das eine negative und/oder neutrale Nettoladung besitzt (anionisches und/oder neutrales Amphiphil).

10. Das Nanoaggregat nach einem der Ansprüche 7 bis 9, in dem das anionische und/oder neutrale Amphiphil aus Sterolen oder Lipiden ausgewählt ist, wie zum Beispiel Cholesterin, Phospholipiden, Lysolipiden, Lysophospholipiden, Sphingolipden oder pegylierten Lipiden mit einer negativen oder neutralen Nettoladung.

11. Das Nanoaggregat nach einem der Ansprüche 7 bis 10, in dem das neutrale Amphiphil Diacylphosphatidylcholin ist.

12. Das Nanoaggregat nach einem der Ansprüche 7 bis 11, das einen Überschuss an positiv geladenen Resten von mindestens etwa 20 % umfasst, bevorzugt von mindestens etwa 30 % und am meisten bevorzugt von mindestens etwa 40 % in der äußeren Molekularschicht.

13. Das Nanoaggregat nach einem der Ansprüche 7 bis 12, das als ein Emulsionströpfchen, eine Mizelle, ein Liposom, ein Nanopartikel oder eine Nanokapsel vorliegt.

14. Das Nanoaggregat nach einem der Ansprüche 7 bis 13, das etwa 0,1 bis etwa 50 Mol% eines Camptothecin-Wirkstoffs oder eines Derivats davon umfasst.

15. Das Nanoaggregat nach einem der Ansprüche 7 bis 14, das weiterhin einen Gefrierschutz, der aus einem Zucker oder einem Alkohol oder einer Kombination davon ausgewählt ist, umfasst, wie zum Beispiel Trehalose, Maltose, Sucrose, Glucose, Lactose, Dextran, Mannitol oder Sorbitol.

16. Pharmazeutische Zubereitung, die eine pharmazeutisch wirksame Menge einer Zusammensetzung mit einer positiven Nettoladung umfasst, welche die Carboxylatform eines Camptothecin-Wirkstoffs umfasst, die mit mindestens einem organischen kationischen Molekül assoziiert ist, eine positive Nettoladung aufweist und in der das Molverhältnis des organischen kationischen Moleküls zum Camptothecin-Wirkstoff mindestens etwa 1:1 beträgt, in der die Zusammensetzung im Wesentlichen frei von der Lactonform des Camptothecins ist oder ein kolloidales Nanoaggregat davon, zusammen mit einem pharmazeutisch verträglichen Träger, Verdünnung und/oder Hilfsstoff.

17. Verfahren zur Herstellung des kolloidalen Nanoaggregats nach einem der Ansprüche 7 bis 15, das die Schritte umfasst
(a) Bereitstellen eines Camptothecin-Wirkstoffs, vorzugsweise als ein Salz,
(b) Assoziieren des Camptothecin-Wirkstoffs in seiner Carboxylatform mit einem kationischen Amphiphil, das eine positive Nettoladung aufweist und wahlweise mit mindestens einem weiteren Amphiphil, das eine positive, negative und/oder neutrale Nettoladung aufweist und
(c) Bilden eines kolloidalen Nanoaggregats.

18. Das Verfahren nach Anspruch 17, in dem Schritt b) und c) das Bilden des Nanoaggregats durch eine Homogenisierung, ein Lipidfilm- oder durch ein Lösungsmittelinjektionsverfahren umfasst.

19. Verwendung einer pharmazeutischen Zubereitung nach Anspruch 16 zur Herstellung eines Medikaments zur Behandlung und/oder Vorbeugung von Krebs, Entzündungskrankheiten, diabetischer Retinopathie, rheumatoider Arthritis, Dermatitis, Psoriasis, Magengeschwüren und Makulardegeneration.

## Revendications

1. Composition comprenant la forme carboxylate d'un médicament à base de camptothécine associée à au moins un amphiphile cationique et/ou un polymère cationique (molécule cationique) possédant une charge nette positive, dans laquelle le rapport molaire entre la molécule cationique et le médicament à base de camptothécine est d'au moins environ 1 : 1, dans laquelle ladite composition est essentiellement exempte de la forme lactone de ladite camptothécine.

2. Composition selon la revendication 1, dans laquelle ledit amphiphile cationique est choisi parmi les lipides, les lysolipides ou les lipides pégylés, contenant de préférence un groupe aminé tertiaire ou un groupe ammonium quaternaire, tel que
la N-[1-(2,3-diacyloxy)propyl]-N,N-diméthylamine ou
le N-[1-(2,3-diacyloxy)propyl]-N,N,N-triméthylammonium.

3. Composition selon l'une quelconque des revendications 1 à 2, dans laquelle ledit polymère cationique est un polyélectrolyte, un acide tel qu'une polyallylamine ou une polyéthylène imine, un sucre polymère ou une polyamine, ayant un poids moléculaire entre environ 5 et environ 500 kDa.

4. Composition selon l'une quelconque des revendications 1 à 3, comprenant en outre au moins un amphiphile anionique et/ou neutre.

5. Composition selon la revendication 4, dans laquelle ledit amphiphile anionique et/ ou neutre est choisi parmi les stérols ou les lipides comme le cholestérol, les phospholipides, les lysolipides, les lysophospholipides, les sphingolipides ou les lipides pégylés, ayant une charge nette négative ou neutre.

6. Composition selon l'une quelconque des revendications 4 à 5, dans laquelle l'amphiphile neutre est la diacylphosphatidylcholine.

7. Nanoagrégat colloïdal comprenant une composition selon l'une quelconque des revendications 1 à 6.

8. Nanoagrégat selon la revendication 7, possédant une charge globale positive.

9. Nanoagrégat selon la revendication 7 ou 8, comprenant en outre au moins un amphiphile qui possède une charge nette négative et/ou neutre (amphiphile anionique et/ou neutre).

10. Nanoagrégat selon l'une quelconque des revendications 7 à 9, dans lequel ledit amphiphile anionique et/ou neutre est choisi parmi les stérols ou les lipides comme le cholestérol, les phospholipides, les lysolipides, les lysophospholipides, les sphingolipides ou les lipides pégylés, ayant une charge nette négative ou neutre.

11. Nanoagrégat selon l'une quelconque des revendications 7 à 10, dans lequel l'amphiphile neutre est la diacylphosphatidylcholine.

12. Nanoagrégat selon l'une quelconque des revendications 7 à 11, comprenant un excès de fragments positivement chargés d'au moins environ 20 %, de préférence au moins environ 30 % et de manière préférée entre toutes d'au moins environ 40 % dans la couche moléculaire externe.

13. Nanoagrégat selon l'une quelconque des revendications 7 à 12, qui est présent sous forme d'une gouttelette d'émulsion, d'une micelle, d'un liposome, d'une nanoparticule ou d'une nanocapsule.

14. Nanoagrégat selon l'une quelconque des revendications 7 à 13, comprenant environ 0,1 à environ 50 % en mole d'un médicament à base de camptothécine ou d'un dérivé de celui-ci.

15. Nanoagrégat selon l'une quelconque des revendications 7 à 14, comprenant en outre un cryoprotecteur qui est choisi parmi un sucre ou un alcool ou une combinaison de ceux-ci, comme le tréhalose, le maltose, le saccharose, le glucose, le lactose, le dextrane, le mannitol ou le sorbitol.

16. Préparation pharmaceutique comprenant une quantité efficace sur le plan pharmaceutique d'une composition comprenant la forme carboxylate d'un médicament à base de camptothécine associée à au moins une molécule cationique organique possédant une charge nette positive, dans laquelle le rapport molaire entre la molécule organique cationique et le médicament à base de camptothécine est d'au moins environ 1 : 1, dans laquelle ladite composition est essentiellement exempte de la forme lactone de ladite camptothécine, ou un nanoagrégat colloïdal de celle-ci, avec un support, diluant et/ou adjuvant pharmaceutiquement acceptable.

17. Procédé de production d'un nanoagrégat colloïdal selon l'une quelconque des revendications 7 à 15, comprenant les étapes suivantes
(a) la fourniture d'un médicament à base de camptothécine, de préférence sous la forme d'un sel,
(b) l'association dudit médicament à base de camptothécine sous sa forme carboxylate avec un amphiphile cationique possédant une charge nette positive et facultativement au moins un amphiphile supplémentaire qui possède une charge nette positive, négative et/ou neutre, et
(c) la formation d'un nanoagrégat colloïdal.

18. Procédé selon la revendication 17, dans lequel l'étape b) et l'étape c) comprennent la formation dudit nanoagrégat par une homogénéisation, un film lipidique ou par une procédure d'injection de solvant.

19. Utilisation d'une préparation pharmaceutique selon la revendication 16, pour produire un médicament destiné à traiter et/ou à prévenir le cancer, les maladies inflammatoires, la rétinopathie diabétique, la polyarthrite rhumatoïde, la dermatite, le psoriasis, les ulcères de l'estomac et la dégénérescence maculaire.
